# EUROPEAN PATENT SPECIFICATION

(11) **EP 1 506 960 B1**
(45) Date of publication and mention of the grant of the patent: **06.04.2011**
(21) Application number: 03254909.9
(22) Date of filing: 07.08.2003
(51) Int. Cl.: C07D 209/08, C07D 209/10, C07D 209/12, C07D 209/30, C07D 401/06, C07D 405/06, C07D 491/04, A61K 31/404, A61P 35/00

(54) **Indole compounds as inhibitors of tubulin polymerisation for the treatment of angiogenesis-related disorders**
Indol-Verbindungen als Inhibitoren der Tubulin-Polymerisation zur Behandlung von angiogenesisbezogenen Erkrankungen
Composés d'indole utilisés comme inhibiteurs de la polymerisation de tubuline pour le traitement de maladies associees a l'angiogenese

(43) Date of publication of application: 16.02.2005
(73) Proprietor: National Health Research Institutes, Taipei, 11529 (TW)
(72) Inventor: Hsieh, Hsing-Pang, Taipei, Taiwan (CN); Chang, Jang-Yang, Taipei, Taiwan 106 (CN); Liou, Jing-Ping, Taipei, Taiwan 106 (CN); Chang, Chun-Wei, 4 Fl. No. 19, Taipei, Taiwan 112 (CN)
(74) Representative: Taylor, Kate Laura

(56) References cited:
- WO-A-01/19794
- WO-A-00/048606
- WO-A-02/036561
- WO-A-02/060872
- US-A- 3 557 142
- POWELL M., SAINSBURY M.: TETRAHEDRON LETTERS, vol. 22, no. 47, 1981, pages 4751-4754, XP009023031

## Description

### BACKGROUND

Cancer treatment can be approached by several modes of therapy, including surgery, radiation, chemotherapy, or a combination of any of these treatments. Among them, chemotherapy is indispensable for inoperable or metastatic forms of cancer.

The microtubule system of eukaryotic cells is an important target for developing anti-cancer agents. More specifically, tubulin polymerization/depolymerization is a popular target for new chemotherapeutic agents. A variety of clinically used compounds (e.g., paclitaxel, epothilone A, vinblastine, combretastatin A-4, dolastatin 10, and colchicine) target tubulin polymerization/depolymerization and disrupt cellular microtubule structures, resulting in mitotic arrest and inhibition of the growth of new vascular epithelial cells. See, e.g., Jordan et al. (1998) Med. Res. Rev. 18: 259-296. Thus, those compounds may have the ability to inhibit excessive angiogenesis, which occurs in diseases such as cancer (both solid and hematologic tumors), cardiovascular diseases (e.g., atherosclerosis), chronic inflammation (e.g., rheutatoid arthritis or Crohn's disease), diabetes (e.g., diabetic retinopathy), macular degeneration, psoriasis, endometriosis, and ocular disorders (e.g., corneal or retinal neovascularization). See, e.g., Griggs et al. (2002) Am. J. Pathol. 160(3): 1097-103.

Take combretastatin A-4 (CA-4) for example. CA-4, isolated by Pettit and coworkers in 1982 (Can. J Chem. 60: 1374-1376), is one of the most potent anti-mitotic agents derived from the stem wood of the South African tree *Combretum caffrum.* This agent shows strong cytotoxicity against a wide variety of human cancer cells, including multi-drug resistant cancer cells. See, e.g., Pettit et al. (1995) J. Med. Chem. 38: 1666-1672; Lin et al. (1989) Biochemistry 28: 6984-6991; and Lin et al. (1988) Mol. Pharmacol. 34: 200-208. CA-4, structurally similar to colchicines, possesses a higher affinity for the colchicine binding site on tubulin than colchicine itself. Pettit et al. (1989) Experientia 45: 209-211. It also has been shown to possess anti-angiogenesis activity. See Pinney *et al.* WO 01/68654A2. The low water-solubility of CA-4 limits its efficacy *in vivo.* See, e.g., Chaplin et al. (1999) Anticancer Research 19: 189-195; and Grosios et al. (1999) Br. J. Cancer 81: 1318-1327.

WO 01 19794 discloses Trimethoxyphenyl substituted indole ligands.

WO02 06872 discloses methods for the synthesis of chemical compounds for screening as potential tubulin polymerization inhibitors. The document also discloses chemical compounds with tubulin polymerization inhibitor activity.

WO 00 048606 discloses the treatment of warm-blooded animals having a tumor or non-malignant hypervascularization, by administering a sufficient amount of a cytotoxic agent formulated into a phosphate prodrug form having substrate specificity for microvessel phosphatases.

Identification of compounds that also target the microtubule system (e.g., tubulin polymerization/depolymerization) can lead to new therapeutics useful in treating or preventing cancer or symptoms associated with cancer.

### SUMMARY

This invention is based on the discovery that indole compounds have anti-cancer activities, and function via targeting the microtubule system (e.g., tubulin polymerization/depolymerization) or others.

In one aspect, this invention features indole compounds of the following formula: 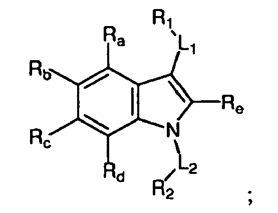 wherein L₁ is C(O); L₂ is a bond; R₁ is 5, 6, or 7-membered aryl or heteroaryl tri-substituted with alkyloxy; R₂ is H, aryl, heteroaryl, halogen, nitro, nitroso, cyano, azide, isothionitro, OR, OC(O)R, OC(O)OR, OC(O)NRR', SO₂R, SO₃R, SO₂NRR' SR, NRR', NRSO₂NR'R", NRSO₂R', NRSO₃R', NRC(O)R', NRC(O)NR'R", NRC(O)OR', NRC(N)NR'R", C(O)OR, C(O)NRR', an amino acid moiety, a polypeptide moiety, a carbohydrate moiety, or a polyethylene glycol (PEG) moiety, in which each of said aryl or heteroaryl, independently is unsubstituted or optionally substituted with halogen, cyano, nitro, hydroxyl, amino, mercapto, alkyl, alkenyl, alkynyl, aryl, heteroaryl, cyclyl, heterocyclyl, alkyloxy, aryloxy, alksulfanyl, arylsulfanyl, alkylamino, arylamino, dialkylamino, diarylamino, alkylcarbonyl, arylcarbonyl, heteroarylcarbonyl, alkylcarboxyl, arylcarboxyl, heteroarylcarboxyl, alkyloxycarbonyl, aryloxycarbonyl, heteroaryloxycarbonyl, alkylcarbamido, arylcarbamido, heterocarbamido, alkylcarbamyl, arylcarbamyl, heterocarbamyl; each of Rₐ, R_{b}, R_{c}, and R_{d}, independently, is R, halogen, nitro, nitroso, cyano, azide, isothionitro, OR, OC(O)R, OC(O)OR, OC(O)NRR', SO₂R, SO₃R, SO₂NRR', SR, NRR', NRSO₂NR'R" NRSO₂R', NRSO₃R', NRC(O)R', NRC(O)NR'R", NRC(O)OR', NRC(N)NR'R", C(O)R, C(O)OR, C(O)NRR', OP(O)(OR)(OR'), O(CH₂)ₙOP(O)(OR)(OR'), or O(CH₂)ₙ-PEG, or R_{b} and R_{c}, Rₐ and R_{b}, or R_{c} and R_{d} taken together are O(CH₂)ₙO; and Rₑ is H or, C₁-C₆ alkyl, in which said C₁-C₆ alkyl, is unsubstituted or optionally substituted with halogen, cyano, nitro, hydroxyl, amino, mercapto, alkyl, alkenyl, alkynyl, cyclyl, heterocyclyl, alkyloxy, alksulfanyl,alkylamino, dialkylamino, alkylcarbonyl, alkylcarboxyl, alkyloxycarbonyl, alkylcarbamido, heterocarbamido, alkylcarbamyl, heterocarbamyl; in which each of R, R', and R", independently, is H, C₁-C₆ alkyl, C₂-C₆ alkenyl, C₂-C₆ alkynyl, aryl, heteroaryl, cyclyl, or heterocyclyl; n is 1, 2, 3, 4, or 5; and in which each of said C₁-C₆ alkyl, C₂-C₆ alkenyl, C₂-C₆ alkynyl, aryl, heteroaryl, cyclyl, or heterocyclyl, independently is unsubstituted or optionally substituted with halogen, cyano, nitro, hydroxyl, amino, mercapto, alkyl, alkenyl, alkynyl, aryl, heteroaryl, cyclyl, heterocyclyl, alkyloxy, aryloxy, alksulfanyl, arylsulfanyl, alkylamino, arylamino, dialkylamino, diarylamino, alkylcarbonyl, arylcarbonyl, heteroarylcarbonyl, alkylcarboxyl, arylcarboxyl, heteroarylcarboxyl, alkyloxycarbonyl, aryloxycarbonyl, heteroaryloxycarbonyl, alkylcarbamido, arylcarbamido, heterocarbamido, alkylcarbamyl, arylcarbamyl, heterocarbamyl. Note that the left atom shown in any substituted group described above is closest to the indole ring. Also note that when Rₐ, R_{b}, R_{c}, and R_{d} are R, the just-described indole compounds may have different R moieties. The same rule applies to other similar situations.

Referring to the just-described indole compounds, a subset of these compounds is featured by that Rₑ is H or C₁-C₆ alkyl. In these compounds, each of Rₐ, R_{b}, R_{c}, and R_{d}, independently, can be H, alkyloxy, alkyl, or halogen (e.g., R_{c} is alkyloxy, alkyl, or halogen, and each of Rₐ, R_{b}, and R_{d} is H). R₁ can be 3,4,5-trimethoxylphenyl. In some embodiments, one, two, or three of Rₐ, R_{b}, R_{c} and R_{d} are alkyloxy, alkyl, or halogen. Preferably, one of Rₐ, R_{b}, R_{c}, and R_{d} is alkyloxy, alkyl, or halogen, and the others are H. More preferably, R_{c} is alkyloxy, alkyl, or halogen (e.g., R_{c} is OCH₃, OCH₂CH₃, CH₃, F, or Br), and each of Rₐ, R_{b}, and R_{d} is H. R₂ can be H, OR, C(O)NRR', C(O)OR (e.g., C(O)OC(CH₃)₃, or C(O)OC₆H₅), or SO₂R (e.g., SOCH₃, or SO₂(4-CO₂H-C₆H₄)). In other embodiments, R_{b} and R_{c} taken together are O(CH₂)ₙO, and each of Rₐ and R_{d} is H, in which n is 1 or 2. In other embodiments 5, 6, or 7-member aryl or heteroaryl tri-substituted with 3,4,5-trimethoxylphenyl.

In another aspect, this invention features indole compounds of the formula above, wherein L₁ is C(O); L₂ is a bond; R₁ is 5, 6, or 7-membered aryl or heteroaryl tri-substituted with alkyloxy; R₂ is C₁-C₆ alkyl, C₂-C₆ alkenyl, C₂-C₆ alkynyl, cyclyl, or heterocyclyl, in which each of said C₁-C₆ alkyl, C₂-C₆ alkenyl, C₂-C₆ alkynyl, cyclyl, or heterocyclyl, independently is unsubstituted or optionally substituted with halogen, cyano, nitro, hydroxyl, amino, mercapto, alkyl, alkenyl, alkynyl, aryl, heteroaryl, cyclyl, heterocyclyl, alkyloxy, aryloxy, alksulfanyl, arylsulfanyl, alkylamino, arylamino, dialkylamino, diarylamino, alkylcarbonyl, arylcarbonyl, heteroarylcarbonyl, alkylcarboxyl, arylcarboxyl, heteroarylcarboxyl, alkyloxycarbonyl, aryloxycarbonyl, heteroaryloxycarbonyl, alkylcarbamido, arylcarbamido, heterocarbamido, alkylcarbamyl, arylcarbamyl, heterocarbamyl; each of Rₐ, R_{b}, P_{c}, and R_{d}, independently, is R, halogen, nitro, isothionitro, OR, OC(O)R, OC(O)OR, OC(O)NRR', SO₂R, SO₃R, SO₂NRR', SR, NRSO₂NR'R", NRSO₂R', NRSO₃R', NRC(O)R', NRC(O)NR'R", NRC(O)OR', NRC(N)NR'R", C(O)R, C(O)OR, C(O)NRR', OP(O)(OR)(OR'), O(CH₂)ₙOP(O)(OR)(OR'), or O(CH₂)ₙ-PEG, or R_{b} and R_{c}, Rₐ and R_{b}, or R_{c}, and R_{d} taken together are O(CH₂)ₙO; and Rₑ is H or, C₁-C₆ alkyl, in which said C₁-C₆ alkyl, is unsubstituted or optionally substituted with halogen, cyano, nitro, hydroxyl, amino, mercapto, alkyl, alkenyl, alkynyl, cyclyl, heterocyclyl, alkyloxy, alksulfanyl,alkylamino, dialkylamino, alkylcarbonyl, alkylcarboxyl, alkyloxycarbonyl, alkylcarbamido, heterocarbamido, alkylcarbamyl, heterocarbamyl; in which each of R, R', and R", independently, is H, C₁-C₆ alkyl, C₂-C₆ alkenyl, C₂-C₆ alkynyl, aryl, heteroaryl, cyclyl, or heterocyclyl; n is 1, 2, 3, 4, or 5; and in which each of said C₁-C₆ alkyl, G₂-C₆ alkenyl, C₂-C₆ alkynyl, aryl, heteroaryl, cyclyl, or heterocyclyl, independently is unsubstituted or optionally substituted with halogen, cyano, nitro, hydroxyl, amino, mercapto, alkyl, alkenyl, alkynyl, aryl, heteroaryl, cyclyl, heterocyclyl, alkyloxy, aryloxy, alksulfanyl, arylsulfanyl, alkylamino, arylamino, dialkylamino, diarylamino, alkylcarbonyl, arylcarbonyl, heteroarylcarbonyl, alkylcarboxyl, arylcarboxyl, heteroarylcarboxyl, alkyloxycarbonyl, aryloxycarbonyl, heteroaryloxycarbonyl, alkylcarbamido, arylcarbamido, heterocarbamido, alkylcarbamyl, arylcarbamyl, heterocarbamyl.

Referring to the just-described indole compounds, a subset of these compounds is featured by that Rₑ is H or C₁-C₆ alkyl. In these compounds, each of Rₐ, R_{b}, R_{c}, and R_{d}, independently, can be H, alkyloxy, alkyl, or halogen (e.g., R_{c} is alkyloxy, alkyl, or halogen, and each of Rₐ, R_{b}, and R_{d} is H). R₁ can be 3,4,5-trimethoxylphenyl. In some embodiments, one, two, or three of Rₐ, R_{b}, R_{c}, and R_{d} are alkyloxy, alkyl, or halogen. Preferably, one of Rₐ, R_{b}, R_{c}, and R_{d} is alkyloxy, alkyl, or halogen, and the others are H. More preferably, R_{c} is alkyloxy, alkyl, or halogen (e.g., R_{c} is OCH₃), and each of Rₐ, R_{b}, and R_{d} is H. R₂ can be alkyl, alkenyl, or alkynyl (e.g., CH₃, C₂H₅, CH₂CH=CH₂, CH₂C≡CH, or CH₂-4-pyridinyl). In other embodiments, R_{b} and R_{c} taken together are O(CH₂)ₙO, and each of Rₐ and R_{d} is H, in which n is 1 or 2. In other embodiments 5, 6, or 7-member aryl or heteroaryl tri-substituted with 3,4,5-trimethoxylphenyl.

In still another aspect, this invention features indole compounds of the formula above, wherein L₁ is C(O); L₂ is a bond; R₁ is 5, 6, or 7-membered aryl or heteroaryl tri-substituted with alkyloxy; R₂ is COR"'; each of Rₐ, R_{b}, R_{c}, and R_{d}, independently, is R, halogen, nitro, nitroso, cyano, azide, isothionitro, OR, OC(O)R, OC(O)OR, OC(O)NRR', SO₂R, SO₃R, SO₂NRR' SR, NRR', NRSO₂NR'R", NRSO₂R' NRSO₃R', NRC(O)R', NRC(O)NR'R", NRC(O)OR', NRC(N)NR'R", C(O)R, C(O)OR, C(O)NRR', OP(O)(OR)(OR'), O(CH₂)ₙOP(O)(OR)(OR'), or O(CH₂)ₙ-PEG, or R_{b} and R_{c}, Rₐ and R_{b}, or R_{c} and R_{d} taken together are O(CH₂)ₙO; and Rₑ is H or, C₁-C₆ alkyl, in which said C₁-C₆ alkyl, is unsubstituted or optionally substituted with halogen, cyano, nitro, hydroxyl, amino, mercapto, alkyl, alkenyl, alkynyl, cyclyl, heterocyclyl, alkyloxy, alksulfanyl,alkylamino, dialkylamino, alkylcarbonyl, alkylcarboxyl, alkyloxycarbonyl, alkylcarbamido, heterocarbamido, alkylcarbamyl, heterocarbamyl; in which each of R, R', and R" independently, is H, C₁-C₆ alkyl, C₂-C₆ alkenyl, C₂-C₆ alkynyl, aryl, heteroaryl, cyclyl, or heterocycly]; R"' is H, C₁-C₆ alkyl, C₂-C₆ alkenyl, C₂-C₆ alkynyl, heteroaryl, cyclyl, or heterocycly]; n is 1, 2, 3, 4, or 5; and in which each of said C₁-C₆ alkyl, C₂-C₆ alkenyl, C₂-C₆ alkynyl, aryl, heteroaryl, cyclyl, or heterocyclyl, independently is unsubstituted or optionally substituted with halogen, cyano, nitro, hydroxyl, amino, mercapto, alkyl, alkenyl, alkynyl, aryl, heteroaryl, cyclyl, heterocyclyl, alkyloxy, aryloxy, alksulfanyl, arylsulfanyl, alkylamino, arylamino, dialkylamino, diarylamino, alkylcarbonyl, arylcarbonyl, heteroarylcarbonyl, alkylcarboxyl, arylcarboxyl, heteroarylcarboxyl, alkyloxycarbonyl, aryloxycarbonyl, heteroaryloxycarbonyl, alkylcarbamido, arylcarbamido, heterocarbamido, alkylcarbamyl, arylcarbamyl, heterocarbamyl.

Referring to the just-described indole compounds, a subset of these compounds is featured by that Rₑ is H or C₁-C₆ alkyl. In these compounds, each of Rₐ, R_{b}, R_{c}, and R_{d}, independently, can be H, alkyloxy, alkyl, or halogen (e.g., R_{c} is alkyloxy, alkyl, or halogen, and each of Rₐ, R_{b}, and R_{d} is H). R₁ can be 3,4,5-trimethoxylphenyl. In some embodiments, one, two, or three of Rₐ, R_{b}, R_{c}, and R_{d} are alkyloxy, alkyl, or halogen. Preferably, one of Rₐ, R_{b}, R_{c}, and R_{d} is alkyloxy, alkyl, or halogen, and the others are H. More preferably, R_{c} is alkyloxy, alkyl, or halogen (e.g., R_{c} is OCH₃), and each of Rₐ, R_{b}, and R_{d} is H. R"' is alkyl (e.g., CH₂NRR'), alkenyl (e.g., (E)-CH=CH-C₆H₅), or heteroaryl (e.g., 2-pyridinyl, 3-pyridinyl, 2-furyl, or 2-thienyl). In other embodiments, R_{b} and R_{c} taken together are O(CH₂)ₙO, and each of Rₐ and R_{d} is H, in which n is 1 or 2. In other embodiments 5, 6, or 7-member aryl or heteroaryl tri-substituted with 3,4,5-trimethoxylphenyl.

Unless specifically pointed out, alkyl, alkenyl, alkynyl, aryl, heteroaryl, cyclyl, and heterocyclyl mentioned herein include both substituted and unsubstituted moieties. The term "substituted" refers to one or more substituents (which may be the same or different), each replacing a hydrogen atom. Examples of substituents include, but are not limited to, halogen, cyano, nitro, hydroxyl, amino, mercapto, alkyl, alkenyl, alkynyl, aryl, heteroaryl, cyclyl, heterocyclyl, alkyloxy, aryloxy, alksulfanyl, arylsulfanyl, alkylamino, arylamino, dialkylamino, diarylamino, alkylcarbonyl, arylcarbonyl, heteroarylcarbonyl, alkylcarboxyl, arylcarboxyl, heteroarylcarboxyl, alkyloxycarbonyl, aryloxycarbonyl, heteroaryloxycarbonyl, alkylcarbamido, arylcarbamido, heterocarbamido, alkylcarbamyl, arylcarbamyl, heterocarbamyl, wherein each of alkyl (including alk), alkenyl, aryl, heteroaryl, cyclyl, and heterocyclyl is optionally substituted with halogen, cyano, nitro, hydroxyl, amino, mercapto, alkyl, aryl, heteroaryl, alkyloxy, aryloxy, alkylcarbonyl, arylcarbonyl, alkylcarboxyl, arylcarboxyl, alkyloxycarbonyl, or aryloxycarbonyl.

As used herein, the term "alkyl" refers to a straight-chained or branched alkyl group containing 1 to 6 carbon atoms. Examples of alkyl groups include methyl, ethyl, n-propyl, isopropyl, tert-butyl, and n-pentyl. Similarly, the term "alkenyl" or "alkynyl" refers to a straight-chained or branched alkenyl or alkynyl group containing 2 to 6 carbon atoms.

The term "aryl" refers to a hydrocarbon ring system (mono-cyclic or bi-cyclic) having at least one aromatic ring. Examples of aryl moieties include, but are not limited to, phenyl, naphthyl, and pyrenyl.

The term "heteroaryl" refers to a hydrocarbon ring system (mono-cyclic or bi-cyclic) having at least one aromatic ring which contains at least one heteroatom such as O N, or S as part of the ring system and the reminder being carbon. Examples of heteroaryl moieties include, but are not limited to, furyl, pyrrolyl, thienyl, oxazolyl, imidazolyl, thiazolyl, pyridinyl, pyrimidinyl, quinazolinyl, and indolyl.

The terms "cyclyl" and "heterocyclyl" refer to a partially or fully saturated mono-cyclic or bi-cyclic ring system having from 4 to 14 ring atoms. A heterocyclyl ring contains one or more heteroatoms (e.g., O, N, or S) as part of the ring system and the remainder being carbon. Exemplary cyclyl and heterocyclyl rings are cyclohexane, piperidine, piperazine, morpholine, thiomorpholine, and 1,4-oxazepane.

The terms "an amino acid moiety," "a polypeptide moiety," "a carbohydrate moiety," and "a polyethylene glycol moiety" herein refer to a moiety formed after an amino acid, a polypeptide, a carbohydrate, and a polyethylene glycol couple to an indole compound of the invention, respectively. Examples of a carbohydrate include monosaccharide (e.g., glucose), disaccharide (e.g., sucrose), polysaccharide (e.g., starch and cellulose), and heterosaccharide (e.g., amygdalin). Polyethylene glycols can be linear, branched, or star-shaped.

Set forth below are exemplary compounds of this invention:
(6-methoxy-1H-indol-3-yl)-(3,4,5-trimethoxy-phenyl)-methanone (Compound 1);
(6-methyl-1H-indol-3-yl)-(3,4,5-trimethoxy-phenyl)-methanone (Compound 2);
(6-methoxy-1-pyridin-4-ylmethyl-1H-indol-3-yl)-(3,4,5-trimethoxy-phenyl)-methanone (Compound 3);
(1-allyl-6-methoxy-1H-indol-3-yl)-(3,4,5-trimethoxy-phenyl)-methanone (Compound 4);
[6-methoxy-1-(pyridine-2-carbonyl)-1H-indol-3-yl]-(3,4,5-trimethoxy-phenyl)-methanone (Compound 5);
6-methoxy-3-(3,4,5-trimethoxy-benzoyl)-indole-1-carboxylic acid tert-butyl ester (Compound 6);
(1-methanesulfonyl-6-methoxy-1H-indol-3-yl)-(3,4,5-trimethoxy-phenyl)-methanone (Compound 7);
[6-methoxy-1-(morpholine-4-carbonyl)-1H-indol-3-yl]-(3,4,5-trimethoxy-phenyl)-methanone (Compound 8);
[6-methoxy-1-(2-piperidin-1-yl-ethyl)-1H-indol-3-yl]-(3,4,5-trimethoxy-phenyl)-methanone (Compound 9);
(6-methoxy-1-prop-2-ynyl-1H-indol-3-yl)-(3,4,5-trimethoxy-phenyl)-methanone (Compound 10);
6-methoxy-3-(3,4,5-trimethoxy-benzoyl)-indole-1-carboxylic acid dimethylamide (Compound 11);
1-[6-methoxy-3-(3,4,5-trimethoxy-benzoyl)-indol-1-yl]-3-phenyl-propenone (Compound 12);
6-methoxy-3-(3,4,5-trimethoxy-benzoyl)indole-1-carboxylic acid phenyl ester (Compound 13);
[1-(5-dimethylamino-naphthalene-1-sulfonyl)-6-mehoxy-1H-indol-3-yl]-(3,4,5-trimethoxy-phenyl)-methanone (Compound 14);
[1-(2-dimethylamino-ethyl)-6-methoxy-1H-indol-3-yl]-3,4,5-trimethoxy-phenyl)-methanone (Compound 15);
(6-metboxy-1-methyl-1H-indol-3-yl)-(3,4,5-trimethoxy-phenyl)-methanone (Compound 16);
[1-(2-amino-ethyl)-6-methoxy-1H-indol-3-yl]-(3,4,5-trimethoxy-phenyl)-methanone (Compound 17);
[1-(furan-2-carbonyl)-6-methoxy-1H-indol-3-yl]-(3,4,5-trimethoxy-phenyl)-methanone (Compound 18);
(1-ethyl-6-methoxy-1H-indol-3-yl)-(3,4,5-trimethoxy-phenyl)-methanone (Compound 19);
[6-methoxy-1-(2-morpholin-4-yl-ethyl)-1H-indol-3-yl]-(3,4,5-trimethoxy-phenyl)-methanone (Compound 20);
[1-(4-chloro-benzyl)-6-methoxy-1H-indol-3-yl]-(3,4,5-trimethoxy-phenyl)-methanone (Compound 21);
(1-benzyl-6-methoxy-1H-indol-3-yl)-(3,4,5-trimethoxy-phenyl)-methanone (Compound 22);
(6-fluoro-1H-indol-3-yl)-(3,4,5-trimethoxy-phenyl)-methanone (Compound 23);
(6-bromo-1H-indol-3-yl)-(3,4,5-trimethoxy-phenyl)-methanone (Compound 24);
(4,5,6-trimethoxy-1H-indol-3-yl)-(3,4,5-trimethoxy-phenyl)-methanone (Compound 25);
(5,6-dimethoxy-1H-indol-3-yl)-(3,4,5-trimethoxy-pbenyl)-methanone (Compound 34);
(1,6-dimethyl-1H-indol-3-yl)-(3,4,5-trimethoxy-phenyl)-methanone (Compound 36);
(1-ethyl-6-methyl-1H-indol-3-yl)-(3,4,5-trimethoxy-phenyl)-methanone (Compound 37);
(1-allyl-6-methyl-1H-indol-3-yl)-(3,4,5-trimethoxy-phenyl)-methanone (Compound 38);
(5-ethyl-5H-[1,3]dioxolo[4,5-f]indol-7-yl)-(3,4,5-trimethoxy-phenyl)-methanone (Compound 39);
(5-methyl-5H-[1,3]dioxolo[4,5-f]indol-7-yl)-(3,4,5-trimethoxy-phenyl)-methanone (Compound 40);
(5-allyl-5H-[1,3]dioxolo[4,5-f]indol-7-yl)-(3,4,5-trimethoxy-phenyl)-methanone (Compound 41);
(6-methoxy-2-methyl-1H-indol-3-yl)-(3,4,5-trimethoxy-phenyl)-methanone (Compound 42);
(6-ethoxy-1H-indol-3-yl)-(3,4,5-trimethoxy-phenyl)-methanone (Compound 44);
(7-methoxy-1H-indol-3-yl)-(3,4,5-trimethoxy-phenyl)-methanone (Compound 45);
(4-methoxy-1H-indol-3-yl)-(3,4,5-trimethoxy-phenyl)-methanone (Compound 46);
(5-methoxy-4-methyl-1H-indol-3-yl)-(3,4,5-trimethoxy-phenyl)-methanone (Compound 47);
(4,7-dimethoxy-1H-indol-3-yl)-(3,4,5-trimethoxy-phenyl)-methanone (Compound 48);
(4,6-dimethoxy-1H-indol-3-yl)-(3,4,5-trimethoxy-phenyl)-methanone (Compound 49);
(5,7-dimethoxy-1H-indol-3-yl)-(3,4,5-trimethoxy-phenyl)-methanone (Compound 50);
{6-methoxy-1-[4-(4-nitro-phenyl)-furan-2-ylmethyl]-1H-indol-3-yl}-(3,4,5-trimethoxy-phenyl)-methanone (Compound 51);
(6-hydroxy-1H-indol-3-yl)-(3,4,5-trimethoxy-phenyl)-methanone (Compound 52);
[1-(2-dimethylamino-ethyl)-4,5,6-trimethoxy-1H-indol-3-yl]-(3,4,5-trimethoxy-phenyl)-methanone (Compound 54),
(6-propoxy-1H-indol-3-yl)-(3,4,5-trimethoxy-phenyl)-methanone (Compound 55);
(6-isopropoxy-1H-indol-3-yl)-(3,4,5-trimethoxy-phenyl)-methanone (Compound 56);
(3,5-dimethoxy-phenyl)-(6-methoxy-1H-indol-3-yl)-methanone (Compound 57);
(3,4-dimethoxy-phenyl)-(6-methoxy-1H-indol-3-yl)-methanone (Compound 58);
[6-(3-morpholin-4-yl-propoxy)-1H-indol-3-yl]-(3,4,5-trimethoxy-phenyl)-methanone (Compound 60);
(6-{2-[2-(2-hydroxy-ethoxy)-ethoxy]-ethoxy}-1H-indol-3-yl)-(3,4,5-trimethoxy-phenyl)-methanone (Compound 61);
4-[6-methoxy-3-(3,4,5-trimethoxy-benzoyl)-indole-1-sulfonyl]-benzoic acid (Compound 62);
(5H-[1,3]dioxolo[4,5-f]indol-7-yl)-(3,4,5-trimethoxy-phenyl)-methanone (Compound 63)
{2-[6-methoxy-3-(3,4,5-trimethoxy-benzoyl)-indol-1-yl]-2-oxo-ethyl}-carbamic acid 9H-fluoren-9-yl-methyl ester (Compound 64);
[6-methoxy-1-(pyridine-3-carbonyl)-1H-indol-3-yl]-(3,4,5-trimethoxy-phenyl)-methanone (Compound 65); and
[6-methoxy-1-(thiophene-2-carbonyl)-1H-indol-3-yl]-(3,4,5-trimethoxy-phenyl)-methanone (Compound 66).

Also described herein is a pharmaceutical composition that contains a pharmaceutically acceptable carrier and an effective amount of at least one of the indole compounds described above.

A further aspect of this invention relates to the indole compounds as described above for use in a method for treating cancer, e.g., carcinoma or sarcoma.

The invention includes the just described indole compounds for use in inhibiting tubulin polymerization.

The invention includes the just described indole compounds for use in treating an angiogenesis-related disorder.

The invention comprises use of one or more the just described indole compounds in the manufacture of a medicament for inhibiting cellular proliferation for inducing the killing of hyperproliferation cells.

All of the indole compounds described above include the compounds themselves, as well as their salts . The salts, for example, can be formed between a positively charged substituent (e.g., amino) on a compound and an anion. Suitable anions include, but are not limited to, chloride, bromide, iodide, sulfate, nitrate, phosphate, citrate, methanesulfonate, trifluoroacetate, and acetate. Likewise, a negatively charged substituent (e.g., carboxylate) on a compound can form a salt with a cation. Suitable cations include, but are not limited to, sodium ion, potassium ion, magnesium ion, calcium ion, and an ammonium cation such as teteramethylammonium ion. Examples of prodrugs include esters and other pharmaceutically acceptable derivatives, which, upon administration to a subject, are capable of providing the indole compounds described above (see Goodman and Gilman's, The Pharmacological basis of Therapeutics, 8th ed., McGraw-Hill, Int. Ed. 1992, "Biotransformation of Drugs").

In addition, some of the just-described indole compounds have one or more double bonds, or one or more asymmetric centers. Such compounds can occur as racemates, racemic mixtures, single enantiomers, individual diastereomers, diastereomeric mixtures, and cis- or trans- or *E*- or *Z*- double bond isomeric forms.

Further, the aforementioned indole compounds also include their *N*-oxides. The term "*N*-oxides" refers to one or more nitrogen atoms, when present in an indole compound, are in *N*-oxide form, i.e., N→O.

Combinations of substituents and variables envisioned by this invention are only those that result in the formation of stable indole compounds without departing from the spirit and scope thereof. The term "stable", as used herein, refers to compounds which possess stability sufficient to allow manufacture and which maintains the integrity of the compound for a sufficient period of time to be useful for the purposes detailed herein (e.g., treating cancer).

Also within the scope of this invention are a composition containing one or more of the indole compounds described above for use in treating diseases or disorders described above, and the use of such a composition for the manufacture of a medicament for the aforementioned treatment.

Other features or advantages of the present invention will be apparent from the following detailed description of several embodiments, and also from the appending claims.

### DETAILED DESCRIPTION

The indole compounds described above can be prepared by methods well known in the art, as well as by the synthetic routes disclosed herein. For example, as shown in Scheme 1 below, one can couple an indole compound with an acyl chloride. The 6-position of the starting indole compound can be alkyloxy, e.g., OCH₃. The 2, 4, 5, and 7-positions of the starting indole may be substituted. The product of the coupling reaction, referred to simply as an "indol-3-yl-aryl-methanone" for brevity, can be converted to a 1-substituted-indol-3-yl-aryl-methanone by coupling the indol-3-yl-aryl-methanone with a halide, e.g., RₑCOCl, RₑCH₂Cl, or RₑSO₂Cl. Alternatively, the indol-3-yl-aryl-methanone can be reduced to an indol-3-yl-aryl-methane, which can be further reacted with a halide to produce a 1-substituted, indol-3-yl-aryl-methane. Once again, although the 2, 4, 5, and 7-positions of the starting indole compound may be substituted, the compounds are referred to as 1-substituted-indol-3-yl-aryl-methane for brevity.

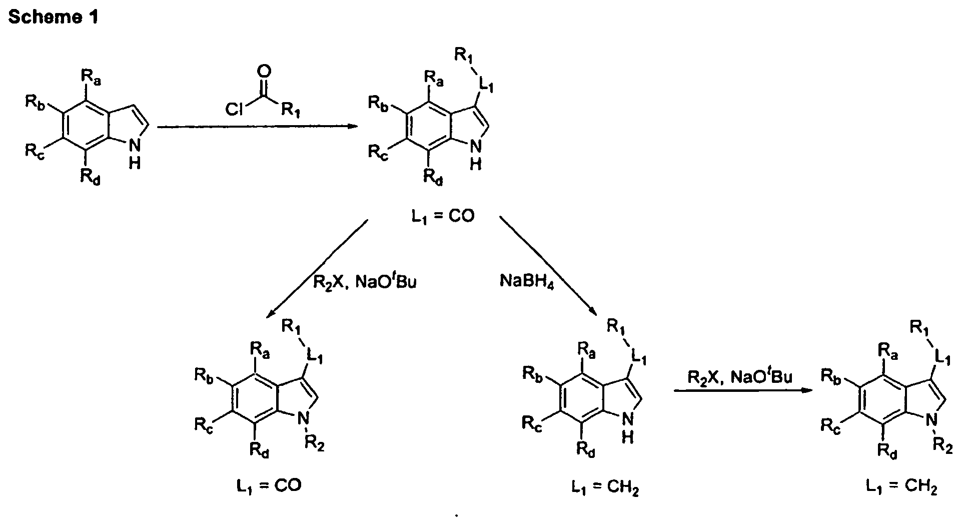

In another example, as shown in Scheme 2 below, one can couple an indole compound with an acyl chloride in the presence of a base (e.g., NaO'Bu). The 5-position of the starting indole can be alkyloxy, e.g., OCH₃. The 2, 4, 6, and 7-positions of the indole may be H, or substituted. The coupling reaction produces an indol-1-yl-aryl-methanone of interest, which can be reduced to an indol-1-yl-aryl-methane.

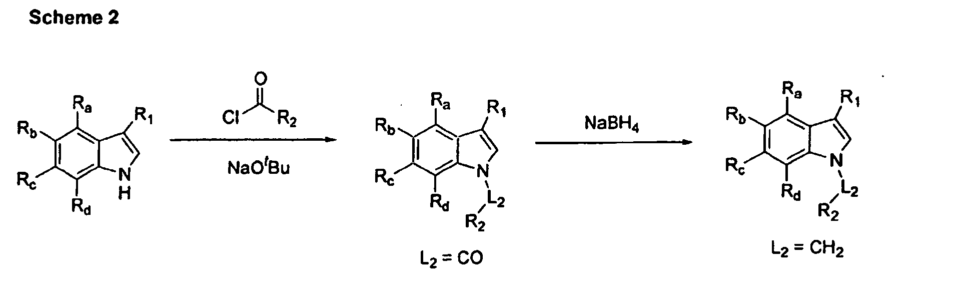

If preferred, indole compounds having other types of L₁ or L₂ can be prepared by similar coupling reactions. See the specific examples below.

An indole compound of the invention can also be prepared by the synthetic methods described above with a suitable derivative (e.g., an acyl chloride derivative) of an amino acid, a polypeptide, a carbohydrate, or a polyethylene glycol.

The chemicals used in the above-described synthetic routes may include, for example, solvents, reagents, catalysts, and protecting group and deprotecting group reagents. The methods described above may also additionally include steps, either before or after the steps described specifically herein, to add or remove suitable protecting groups in order to ultimately allow synthesis of the indole compounds. In addition, various synthetic steps may be performed in an alternate sequence or order to give the desired compounds. Synthetic chemistry transformations and protecting group methodologies (protection and deprotection) useful in synthesizing applicable indole compounds are known in the art and include, for example, those described in R. Larock, Comprehensive Organic Transformations, VCH Publishers (1989); T.W. Greene and P.G.M. Wuts, Protective Groups in Organic Synthesis, 3rd Ed., John Wiley and Sons (1999); L. Fieser and M. Fieser, Fieser and Fieser's Reagents for Organic Synthesis, John Wiley and Sons (1994); and L. Paquette, ed., Encyclopedia of Reagents for Organic Synthesis, John Wiley and Sons (1995) and subsequent editions thereof.

Details of synthesis of Compounds 1-59 of this invention are described in Examples 1-59, respectively.

An indole compound thus obtained can be further purified by flash column chromatography, high performance liquid chromatography, or crystallization.

Also within the scope of this invention is a pharmaceutical composition that contains an effective amount of at least one indole compound of the present invention and a pharmaceutically acceptable carrier.

As used herein, the term "treating" or "treatment" is defined as the application or administration of a composition including an indole compound to a subject, who has a disorder (e.g., cancer), a symptom of the disorder, a disease or disorder secondary to the disorder, or a predisposition toward the disorder, with the purpose to cure, alleviate, relieve, remedy, or ameliorate the disorder, the symptom of the disorder, the disease or disorder secondary to the disorder, or the predisposition toward the disorder. "An effective amount" refers to an amount of an indole compound which confers a therapeutic effect on the treated subject. The therapeutic effect may be objective (i.e., measurably by some tests or markers) or subjective (i.e., a subject gives an indication of or feels an effect). The interrelationship of dosages for animals and humans (based on milligrams per meter squared of body surface) is described in Freireich et al., (1966) Cancer Chemother Rep 50: 219. Body surface area may be approximately determined from height and weight of the patient. See, e.g., Scientific Tables, Geigy Pharmaceuticals, Ardley, N.Y., 1970, 537. An effective amount of the indole compounds can range from about 0.1 mg/Kg to about 1000 mg/Kg. Effective doses will also vary, as recognized by those skilled in the art, depending on the types of tumors treated, route of administration, excipient usage, and the possibility of co-usage with other therapeutic treatments such as use of other anti-cancer agents or radiation therapy. Examples of the other anti-cancer agents are paclitaxel, docitaxel, doxorubicin, daunorubicin, epirubicin, fluorouracil, melphalan, cis-platin, carboplatin, cyclophosphamide, mitomycin C, methotrexate, mitoxantrone, vinblastine, vincristine, ifosfamide, teniposide, etoposide, bleomycin, leucovorin, cytarabine, dactinomycin, interferon alpha, streptozocin, prednisolone, procarbazine, irinotecan, topotecan, colony stimulating factor, granulocyte macrophage colony stimulating factor, 1,3-bis-2-chloroethyl-1-nitroso-urea, and imatinib mesylate.

As used herein, the terms "cancer" and "hyperproliferative" refer to cells having the capacity for autonomous growth, i.e., an abnormal state or condition characterized by rapidly proliferating cell growth. Hyperproliferative disease states may be categorized as pathologic, i.e., characterizing or constituting a disease state, or may be categorized as non-pathologic, i.e., a deviation from normal but not associated with a disease state. The term is meant to include all types of cancerous growths or oncogenic processes, metastatic tissues or malignantly transformed cells, tissues, or organs, irrespective of histopathologic type or stage of invasiveness. "Pathologic hyperproliferative" cells occur in disease states characterized by malignant tumor growth. Examples of non-pathologic hyperproliferative cells include proliferation of cells associated with wound repair.

Examples of cellular proliferative and/or differentiative disorders include cancer, e.g., carcinoma, sarcoma, or metastatic disorders. The indole compounds described above are useful for the treatment of disease caused or exascerbated by cell proliferation. As cell proliferation inhibitors, these compounds are useful in the treatment of both primary and metastatic solid tumors and carcinomas of the breast, colon, rectum, lung, oropharynx, hypopharynx, esophagus, stomach, pancreas, liver, gallbladder, bile ducts, small intestine, urinary tract including kidney, bladder and urothelium, female genital tract including cervix, uterus, ovaries, choriocarcinoma, and gestational trophoblastic disease, male genital tract including prostate, seminal vesicles, testes, and germ cell tumors, endocrine glands including thyroid, adrenal, and pituitary, skin including hemangiomas, melanomas, sarcomas arising from bone or soft tissues including Kaposi's sarcoma, tumors of the brain, nerves, and eyes, meninges including astrocytomas, gliomas, glioblastomas, retinoblastomas, neuromas, neuroblastomas, Schwannomas and meningiomas, solid tumors arising from hematopoietic malignancies including leukemias and chloromas, plasmacytomas, plaques, tumors of mycosis fungoides, cutaneous T-cell lymphoma/leukemia, lymphomas including Hodgkin's and non-Hodgkin's lymphomas, prophylaxis of autoimmune diseases including rheumatoid, immune and degenerative arthritis, ocular diseases including diabetic retinopathy, retinopathy of prematurity, corneal graft rejection, retrolental fibroplasia, neovascular glaucoma, rubeosis, retinal neovascularization due to macular degeneration, hypoxia, abnormal neovascularization conditions of the eye, skin diseases including psoriasis, blood vessel diseases including hemagiomas and capillary proliferation within atherosclerotic plaques, Osler-Webber Syndrome, myocardial angiogenesis, plaque neovascularization, telangiectasia, hemophiliac joints, angiofibroma, and wound granulation. In addition, cancer can be a drug resistance phenotype wherein cancer cells express P-glycoprotein, multidrug resistance-associated proteins, lung cancer resistance-associated proteins, breast cancer resistance proteins, or other proteins associated with resistance to anti-cancer drugs.

The term "angiogenesis" refers to the growth of new blood vessels - an important natural process occurring in the body. In many serious diseases states, the body loses control over angiogenesis. Angiogenesis-dependent diseases result when new blood vessels grow excessively. Examples of angiogenesis-related disorders include cardiovascular diseases (e.g., atherosclerosis), chronic inflammation (e.g., rheutatoid arthritis or Crohn's disease), diabetes (e.g., diabetic retinopathy), macular degeneration, psoriasis, endometriosis, and ocular disorders (e.g., corneal or retinal neovascularization).

The above-described pharmaceutical composition can be administered orally, parenterally, by inhalation spray, topically, rectally, nasally, buccally, vaginally or via an implanted reservoir. The term "parenteral" as used herein includes subcutaneous, intracutaneous, intravenous, intramuscular, intraarticular, intraarterial, intrasynovial, intrasternal, intrathecal, intralesional and intracranial injection or infusion techniques.

A sterile injectable composition, e.g., a sterile injectable aqueous or oleaginous suspension, can be formulated according to techniques known in the art using suitable dispersing or wetting agents (such as Tween 80) and suspending agents. The sterile injectable preparation can also be a sterile injectable solution or suspension in a non-toxic parenterally acceptable diluent or solvent, for example, as a solution in 1,3-butanediol. Among the acceptable vehicles and solvents that can be employed are mannitol, water, Ringer's solution and isotonic sodium chloride solution. In addition, sterile, fixed oils are conventionally employed as a solvent or suspending medium (e.g., synthetic mono- or diglycerides). Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions can also contain a long-chain alcohol diluent or dispersant, or carboxymethyl cellulose or similar dispersing agents. Other commonly used surfactants such as Tweens or Spans or other similar emulsifying agents or bioavailability enhancers which are commonly used in the manufacture of pharmaceutically acceptable solid, liquid, or other dosage forms can also be used for the purposes of formulation.

A composition for oral administration can be any orally acceptable dosage form including, but not limited to, capsules, tablets, emulsions and aqueous suspensions, dispersions and solutions. In the case of tablets for oral use, carriers which are commonly used include lactose and corn starch. Lubricating agents, such as magnesium stearate, are also typically added. For oral administration in a capsule form, useful diluents include lactose and dried corn starch. When aqueous suspensions or emulsions are administered orally, the active ingredient can be suspended or dissolved in an oily phase combined with emulsifying or suspending agents. If desired, certain sweetening, flavoring, or coloring agents can be added. A nasal aerosol or inhalation composition can be prepared according to techniques well-known in the art of pharmaceutical formulation and can be prepared as solutions in saline, employing benzyl alcohol or other suitable preservatives, absorption promoters to enhance bioavailability, fluorocarbons, and/or other solubilizing or dispcrsing agents known in the art. An indole compound-containing composition can also be administered in the form of suppositories for rectal administration.

The carrier in the pharmaceutical composition must be "acceptable" in the sense of being compatible with the active ingredient of the formulation (and preferably, capable of stabilizing it) and not deleterious to the subject to be treated. For example, solubilizing agents such as cyclodextrins, which form specific, more soluble complexes with the indole compounds, or one or more solubilizing agents, can be utilized as pharmaceutical excipients for delivery of the indole compounds. Examples of other carriers include colloidal silicon dioxide, magnesium stearate, cellulose, sodium lauryl sulfate, and D&C Yellow # 10.

The indole compounds can be preliminarily screened for their efficacy in treating cancer by one or more of the following *in vitro* assays.

In one assay, an indole compound is tested for its cytotoxicity on MCF-7 cells (a breast carcinoma cell line). More specifically, cells are incubated with a test compound for 24 hr. The cytotoxic effect can be determined using the MTS (3-(4,5-dimethylthiazol-2-yl)-5-(3-carboxymethoxyphenyl)-2-(4-sulfophenyl)-2*H*-tetrazolium, inner salt) assay method described in Goodwin et al. (1995, J. Immunol. Methods. 179: 95-103). Cytotoxicity of the test compound is expressed in terms of IC₅₀ values (i.e., the concentration of the test compound which achieves a half-maximal inhibition of cell growth).

In another assay, an indole compound is tested for its cytotoxicity in cell cultures and polymerization of tubulin in the absence of GTP. The cytotoxicity is determined using the turbidimetric assay of microtubule protein described by Lopes et al. (1997 Cancer Chemother. Pharmacol. 41: 37-47). Tubulin polymerization is monitored spectrophotometrically by following changes in turbidity as a measure of polymer mass.

The anti-cancer activity of an indole compound can be further assessed using an *in vivo* animal model. See the specific example below.

Without further elaboration, it is believed that the above description has adequately enabled the present invention. The following specific embodiments are, therefore, to be construed as merely illustrative.

### Example 1. Synthesis of Compound 1: 6-methoxy-1H-indol-3-yl)-3,4,5-trimethoxy-phenyl)-methanone

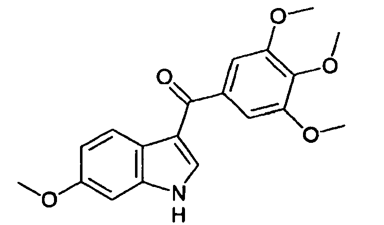

To a mixture of 6-methoxyindole (0.3 g, 2.03 mmol) and anhydrous ZnCl₂ (0.56 g, 4.07 mmol) in CH₂Cl₂ (10 mL), EtMgBr (0.9 ml, 3 M) was added over 10 min at room temperature. The obtaincd suspension was stirred for 1 hr. To the suspension, the solution of 3,4,5-trimethoxybenzoyl chloride/CH₂Cl₂ (10 ml) was added dropwisely during 5 min for a coupling reaction. The reaction mixture continued stirring for another 1 hr and AlCl₃ (0.27 g, 2.03 mmol) was added. The resultant thick mixture was vigorously stirred for 5 hr while monitoring by TLC (EtOAc : *n*-hexane = 1 : 1). The reaction was quenched with H₂O (10 ml) and extracted with CH₂Cl₂ (10 mL x 3). The combined extracts was dried by MgSO₄, and evaporated to give a brown oil which was chromatographed (silica gel; EtOAc : n-hexane = 1 : 1) to afford Compound 1 (0.5 g, 72%) as a white solid.
¹H NMR (CDCl₃), A (ppm): 3.77 (s, 3H), 3.83 (s, 3H), 3.92 (s, 6H), 6.85 (d, *J*= 2.1 Hz, 1H), 6.93 (dd, *J*= 8.9, 2.4 Hz, 1H), 7.08 (s, 2H), 7.59 (s, 1H), 8.22 (d, *J*= 8.7 Hz, 1H), 9.80 (br, 1H, NH).
MS (EI): m/z 342 (M+H).

### Example 2. Synthesis of Compound 2: (6-methyl-1H-indol-3-yl)-(3,4,5-trimethoxy-phenyl)-methanone

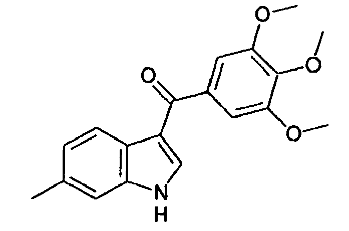

Compound 2 was prepared in a similar manner as described in Example 1.
¹H NMR (CDCl₃), A (ppm): 2.45 (s, 3H), 3.85 (s, 6H), 3.92 (s, 6H), 7.09 (s, 2H), 7.13 (dd, *J =* 8.4, 0.9 Hz, 1H), 7.20 (d, *J* = 0.6 Hz, 1H), 7.63 (d, *J* = 2.7 Hz, 1H), 8.22 (d, *J =* 8.1 Hz, 1H), 9.36 (br, 1H, NH).
MS (EI): m/z 326 (M+H).

### Example 3. Synthesis of Compound 3: (6-methoxy-1-pyridin-4-ylmethyl-1H-indol-3-yl)-(3,4,5-trimethoxy-phenyl)-methanone

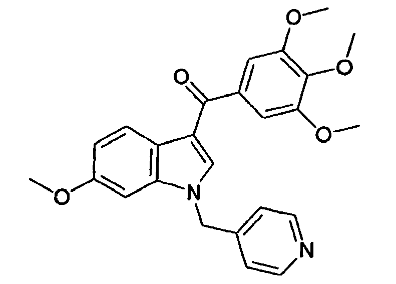

To a stirred solution of Compound 1 (0.07 g, 0.20 mmol), NaOtBu (0.08 g, 0.82 mmol), and 4-picoyl chloride hydrochloride (0.06 g, 0.41 mmol) in THF (10 mL) was heated to reflux. After 15 hr, the reaction mixture was evaporated, and the residue was extracted with CH₂Cl₂ (10 mL x 3). The combined extracts were dried by MgSO₄ and evaporated to give a yellow oil which was chromatographied by silica gel (EtOAc : n-hexane = 2 : 1) to afford Compound 3 (0.10 g, 83%) as a white solid.
¹H NMR (CDCl₃), A (ppm): 3.80 (s, 3H), 3.86 (s, 6H), 3.90 (s, 3H), 5.42 (s, 2H), 6.64 (d, *J* = 2.1 Hz, 1H), 7.00 (dd, *J* = 8.7, 2.1 Hz, 1H), 7.08 (s, 2H), 7.10-7.13 (m, 2H), 7.59 (s, 1H), 8.27 (d, *J* = 9.0 Hz, 1H).
MS (EI): m/z 433 (M+H).

### Example 4. Synthesis of Compound 4: (1-allyl-6-methoxy-1H-indol-3-yl)-(3,4,5-trimethoxy-phenyl)-methanone

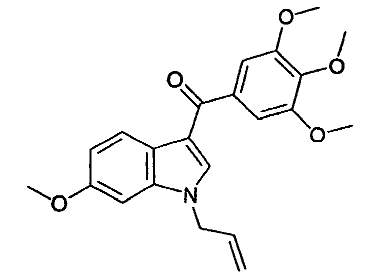

Compound 4 was prepared in a similar manner as described in Example 3.
¹H NMR (CDCl₃), A (ppm): 3.85 (s, 3H), 3.87 (s, 6H), 3.90 (s, 3H), 4.69-4.71 (m, 2H), 5.12-5.28 (m, 2H), 5.94-6.03 (m, 1H), 6.79 (d, *J =* 2.4 Hz, 1H), 6.95 (dd, *J =* 8.7, 2.4 Hz, 1H), 7.07 (s, 2H), 7.51 (s, 1H), 8.23 (d, *J* = 8.7 Hz, 1H).
MS (EI): m/z 382 (M+H).

### Example 5. Synthesis of Compound 5: [6-methoxy-1-(pyridine-2-carbonyl)-1H-indol-3-yl]-(3,4,5-trimethoxy-phenyl)-methanone

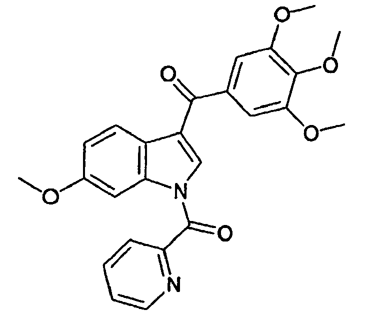

To a solution of Compound 1 (0.1 g, 0.29 mmol) in THF (15 mL) was added NaOtBu (0.11 g, 1.17 mmol) in a portion and stirred at room temperature for 15 min. The resulting dark green mixture was added picolinoyl chloride hydrochloride (0.1 g, 0.58 mmol) and then kept stirring at room temperature. After 15 hr, the reaction mixture was evaporated, and the residue was extracted with CH₂Cl₂ (10 mL x 3). The combined extracts were dried by MgSO₄ and evaporated to get a yellow oil which was chromatographed by silica gel (EtOAc : n-hexane = 1 : 1) to afford Compound 5 (0.11 g, 90%) as a white solid.
¹H NMR (CDCl₃), A (ppm): 3.94 (s, 12H), 7.09 (dd, *J*= 8.7, 2.4 H₂, 1H), 7.24 (s, 2H), 7.53-7.58 (m, 1H), 7.95-8.00 (m, 1H), 8.16-8.19 (m, 3H), 8.60 (s, 1H), 8.66-8.69 (m, 1H).
MS (EI): m/z 455 (M+H).

### Example 6. Synthesis of Compound 6: 6-methoxy-3-(3,4,5-trimethoxy-benzoyl)-indole-1-carboxylic acid tert-butyl ester

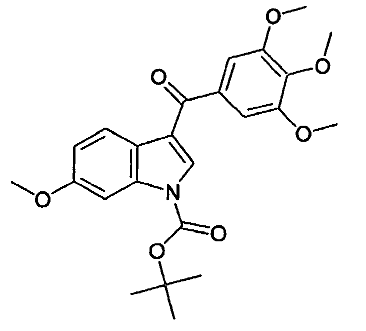

Compound 6 was prepared in a similar manner as described in Example 5.
¹H NMR (CDCl₃), A (ppm): 1.68 (s, 9H), 3.89 (s, 3H), 3.90 (s, 6H), 3.94 (s, 3H), 7.00 (dd, *J =* 8.9, 2.4 Hz, 1H), 7.15 (s, 2H), 7.73 (d, *J =* 2.1 Hz, 1H), 7.99 (s, 1H), 8.13 (d, *J* = 8.7 Hz, 1H).
MS (EI): m/z 442 (M+H).

### Example 7. Synthesis of Compound 7: (1-methanesulfonyl-6-methoxy-1H-indol-3-yl)-(3,4,5-trimethoxy-phenyl)-methanone

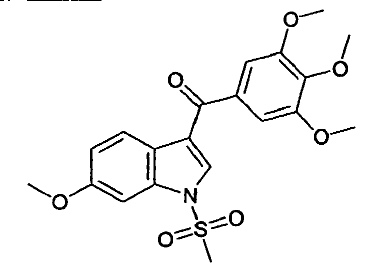

Compound 7 was prepared in a similar manner as described in Example 5.
¹H NMR (CDCl₃), A (ppm): 3.22 (s, 3H), 3.92 (s, 9H), 3.96 (s, 3H), 7.09 (dd, *J*= 9.0, 2.4 Hz, 1H), 7.14 (s, 2H), 7.43 (d, *J* = 2.4 Hz, 1H), 7.84 (s, 1H), 8.20 (d, *J* = 9.0 Hz, 1H).
MS (EI): m/z 420 (M+H).

### Example 8. Synthesis of Compound 8: [6-methoxy-1-(morpholinc-4-carbonyl)-1H-indol-3-yl]-(3,4,5-trimethoxy-phenyl)-methanone

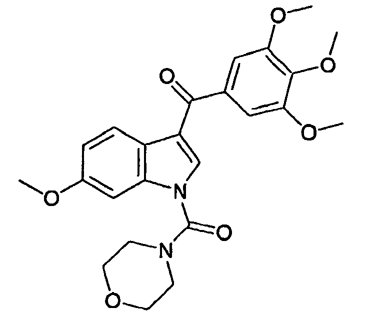

Compound 8 was prepared in a similar manner as described in Example 5.
¹H NMR (CDCl₃), A (ppm): 3.58-3.61 (m, 4H), 3.73-3.76 (m, 4H), 3.87 (s, 9H), 3.92 (s, 3H), 6.98 (dd, *J*= 8.9, 2.1 Hz, 1H), 7.10 (s, 2H), 7.12 (d, *J* = 2.1 Hz, 1H ), 7.68 (s, 1H), 8.13 (d, *J* = 9.0 Hz, 1H).
MS (EI): m/z 455 (M+H).

### Example 9. Synthesis of Compound 9: [6-methoxy-1-(2-piperidin-1-yl-ethyl)-1H-indol-3-yl]-(3,4,5-trimethoxy-phenyl)-methanone

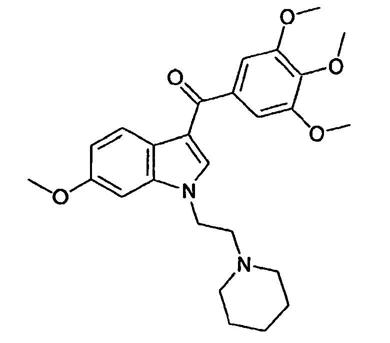

Compound 9 was prepared in a similar manner as described in Example 3.
¹H NMR (CDCl₃), A (ppm): 1.42-1.44 (m, 2H), 1.53-1.60 (m, 4H), 2.44 (t, *J*= 6.6 Hz, 2H), 3.88 (s, 9H), 3.91 (s, 3H), 4.22 (t, *J*= 6.6 Hz, 2H), 6.87 (d, *J*= 2.1 Hz, 1H), 6.96 (dd, *J* = 8.9, 2.1 Hz, 1H), 7.08 (s, 2H), 7.60 (s, 1H), 8.23 (d, *J* = 8.7 Hz, 1H).
MS (EI): m/z 453 (M+H).

### Example 10. Synthesis of Compound 10: (6-methoxy-1-prop-2-ynyl-1H-indol-3-yl)-(3,4,5-trimethoxy-phenyl)-methanone

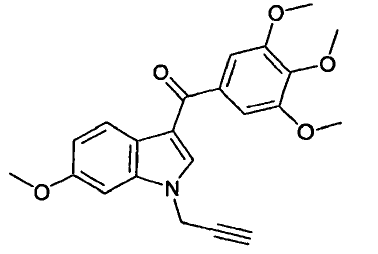

Compound 10 was prepared in a similar manner as described in Example 3.
¹H NMR (CDCl₃), A (ppm): 3.88 (s, 9H), 3.93 (s, 3H), 5.66 (d, *J*= 6.6 Hz, 2H), 6.95-7.16 (m, 5H), 7.58 (s, 1H), 8.20 (d, *J*= 8.7 Hz, 1H).
MS (EI): m/z 380 (M+H).

### Example 11. Synthesis of Compound 11: 6-methoxy-3-(3,4,5-trimethoxy-benzoyl)-indole-1-carboxylic acid dimethylamide

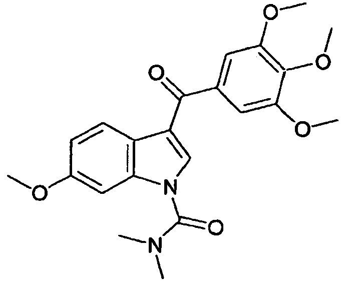

Compound 11 was prepared in a similar manner as described in Example 5.
¹H NMR (CDCl₃), A (ppm): 3.12 (s, 6H), 3.88 (s, 3H), 3.90 (s, 6H), 3.95 (s, 3H), 7.00 (dd, *J* = 8.7, 2.4 Hz, 1H), 7.12 (s, 2H), 7.67 (s, 1H), 8.17 (d, *J* = 8.7 Hz, 1H).
MS (EI): m/z 413 (M+H).

### Example 12. Synthesis of Compound 12: 1-[6-methoxy-3-(3,4,5-trimethoxy-benzoyl)-indol-1-yl]-3-phenyl-propenone

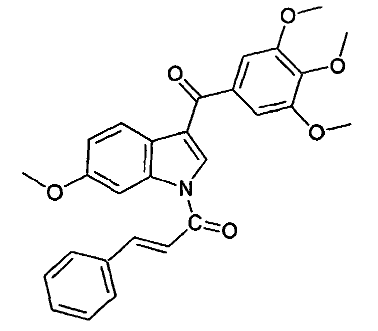

Compound 12 was prepared in a similar manner as described in Example 5.
¹H NMR (CDCl₃), A (ppm): 3.91 (s, 6H), 3.92 (s, 3H), 3.96 (s, 3H), 7.04 (dd, *J*= 8.7, 2.4 Hz, 1H), 7.14-7.20 (m, 3H), 7.41-7.47 (m, 3H), 7.61-7.64 (m, 2H), 8.02-8.11 (m, 4H).
MS (EI): m/z 472 (M+H).

### Example 13. Synthesis of Compound 13: 6-methoxy-3-(3,4,5-trimethoxy-benzoyl)-indole-1-carboxylic acid phenyl ester

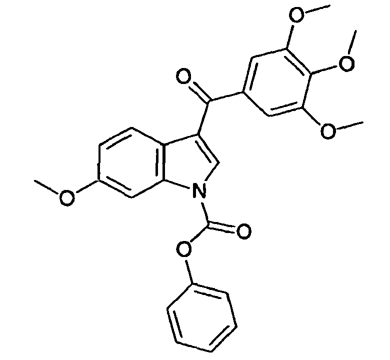

Compound 13 was prepared in a similar manner as described in Example 5.
¹H NMR (CDCl₃), A (ppm): 3.88 (s, 3H), 3.91 (s, 6H), 3.95 (s, 3H), 7.05 (dd, *J*= 8.9, 2.1 Hz, 1H), 7.18 (s, 2H), 7.28-7.37 (m, 3H0, 7.46-7.51 (m, 2H), 7.83 (d, *J* = 2.1 Hz, 1H), 8.14 (s, 1H), 8.15 (d, *J* = 9.0 Hz, 1H).
MS (EI): m/z 462 (M+H).

### Example 14. Synthesis of Compound 14: [1-(5-dimethylamino-naphthalene-1-sulfonyl)-6-methoxy-1H-indol-3-yl]-(3,4,5-trimethoxy-phenyl)-methanone

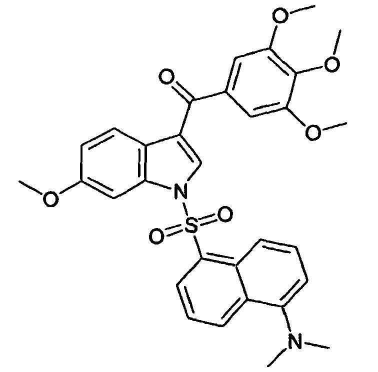

Compound 14 was prepared in a similar manner as described in Example 5.
¹H NMR (CDCl₃), A (ppm): 2.83 (s, 6H), 3.81 (s, 3H), 3.90 (s, 6H), 3.97 (s, 3H), 6.95 (dd, *J*= 8.9, 2.4 Hz, 1H), 7.13 (s, 2H), 7.16 (s, 1H), 7.30 (d, *J*= 2.1 Hz, 1H), 7.48-7.55 (m, 2H), 8.10 (d, *J* = 9.0 Hz, 1H), 8.14 (s, 1H), 8.22-8.31 (m, 2H), 8.60 (d, *J* = 8.7 Hz, 1H).
MS (EI): m/z 575 (M+H).

### Example 15. Synthesis of Compound 15: [1-(2-dimethylamino-ethyl)-6-methoxy-1H-indol-3-yl]-3,4,5-trimethoxy-phenyl)-methanone

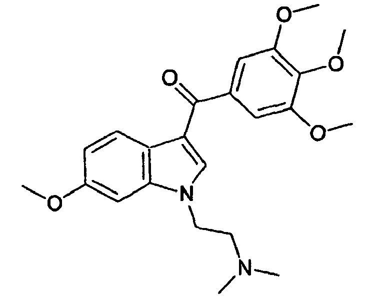

Compound 15 was prepared in a similar manner as described in Example 3.
¹H NMR (CDCl₃), A (ppm): 2.30 (s, 6H), 2.75 (t, *J*= 6.3 Hz, 2H), 3.89 (s, 9H), 3.92 (s, 3H), 4.22 (t, *J=* 6.6 Hz, 2H), 6.86 (d, *J*= 2.4 Hz, 1H), 6.97 (dd, *J*= 8.9, 2.4 Hz, 1H), 7.10 (s, 2H), 7.62 (s, 1H), 8.25 (d, *J* = 9.0 Hz, 1H).
MS (EI): m/z 413 (M+H).

### Example 16. Synthesis of Compound 16: (6-methoxy-1-methyl-1H-indol-3-yl)-(3,4,5-trimethoxy-phenyl)-methanone

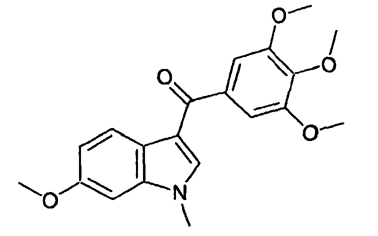

Compound 16 was prepared in a similar manner as described in Example 3.
¹H NMR (CDCl₃), A (ppm): 3.79 (s, 3H), 3.88 (s, 9H), 3.92 (s, 3H), 6.80 (d, *J*= 2.1 Hz, 1H), 6.95 (dd, *J* = 9.0, 2.4 Hz, 1H), 7.07 (s, 2H), 7.47 (s, 1H), 8.23 (d, *J*= 9.0 Hz, 1H).
MS (EI): m/z 356 (M+H).

### Example 17. Synthesis of Compound 17: [1-(2-amino-ethyl)-6-methoxy-1H-indol-3-yl]-(3,4,5-trimethoxy-phenyl)-methanone

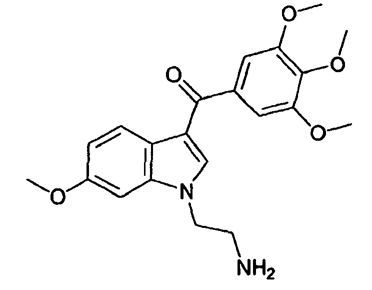

Compound 17 was prepared in a similar manner as described in Example 3.
¹H NMR (CDCl₃), A (ppm): 1.93 (br, 2H, NH₂), 3.15 (br, 2H), 3.89 (s, 9H), 3.90 (s, 3H), 4.19 (t, *J*= 5.1 Hz, 2H), 6.85 (d, *J*= 2.1 Hz, 1H), 6.94 (dd, *J*= 8.7, 2.1 Hz, 1H), 7.07 (s, 2H), 7.62 (s, 1H), 8.21 (d, *J* = 8.4 Hz, 1H).
MS (EI): m/z 385 (M+H).

### Example 18. Synthesis of Compound 18: [1-(furan-2-carbonyl)-6-methoxy-1H-indol-3-yl]-(3,4,5-trimethoxy-phenyl)-methanone

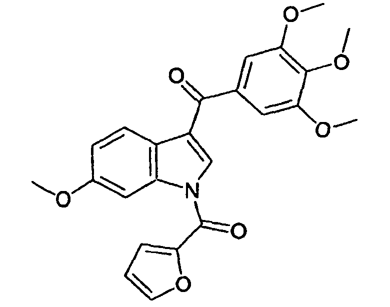

Compound 18 was prepared in a similar manner as described in Example 5.
¹H NMR (CDCl₃), **∧** (ppm): 3.91 (s, 9H), 3.94 (s, 3H), 6.67 (dd, *J*= 3.6, 1.8 Hz, 1H), 7.06 (dd, *J*= 8.7, 2.1 Hz, 1H), 7.20 (s, 2H), 7.52 (dd, *J=* 3.6, 0.9 Hz, 1H), 7.67 *(dd, J=* 1.8,0.9 Hz, 1H0, 8.05 (d, *J*= 2.4 Hz, 1H), 8.12 (d, *J* = 8.7 Hz, 1H), 8.45 (s, 1H).
MS (EI): m/z 436 (M+H).

### Example 19. Synthesis of Compound 19: (1-ethyl-6-methoxy-1H-indol-3-yl)-(3,4,5-trimethoxy-phenyl)-methanone

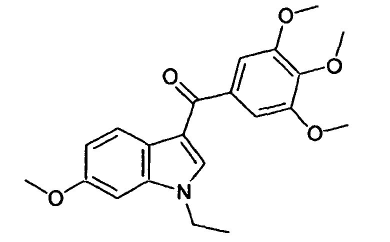

Compound 19 was prepared in a similar manner as described in Example 3.
¹H NMR (CDCl₃), A (ppm): 1.49 (t, *J*= 7.2 Hz, 3H), 3.88 (s, 9H), 3.91 (s, 3H), 4.15 (q, *J* = 7.5 Hz, 2H), 6.82 (d, *J=* 2.4 Hz, 1H), 6.95 (dd, *J=* 9.0, 2.4 Hz, 1H), 7.07 (s, 2H), 7.54 (s, 1H), 8.22 (d, *J* = 9.0 Hz, 1H).
MS (EI): m/z 370 (M+H).

### Example 20. Synthesis of Compound 20: [6-methoxy-1-(2-morpholin-4-yl-ethyl)-1H-indol-3-yl]-(3,4,5-trimethoxy-phenyl)-methanone

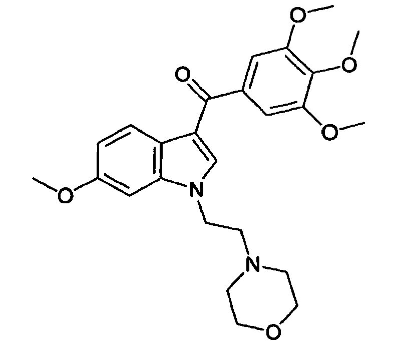

Compound 20 was prepared in a similar manner as described in Example 3.
¹H NMR (CDCl₃), A (ppm): 2.48 (t, *J*= 4.2 Hz, 4H), 2.79 (t, *J*= 6.6 Hz, 2H), 3.66 (t, *J* = 4.5 Hz, 4H), 3.87 (s, 9H), 3.91 (s, 3H), 4.22 (t, *J* = 6.6 Hz, 2H), 6.85 (d, *J* = 2.1 Hz, 1H), 6.95 (dd, *J* = 8.7, 2.4 Hz, 1H), 7.07 (s, 2H), 7.58 (s, 1H), 8.21 (d, *J* = 9.0 Hz, 1H).
MS (EI): m/z 455 (M+H).

### Example 21. Synthesis of Compound 21: [1-(4-chloro-benzyl)-6-methoxy-1H-indol-3-yl]-(3,4,5-trimethoxy-phenyl)-methanone

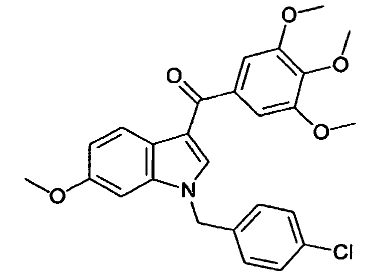

Compound 21 was prepared in a similar manner as described in Example 3.
¹H NMR (CDCl3), A (ppm): 3.80 (s, 3H), 3.81 (s, 6H), 3.89 (s, 3H), 5.24 (s, 2H), 6.73 (d, *J* = 2.1 Hz, 1H), 6.95 (dd, *J*= 8.7, 2.1 Hz, 1H), 7.03 (s, 2H), 7.06 (d, *J=* 6.9 Hz, 2H), 7.26 (d, *J* = 6.6 Hz, 2H), 7.49 (s, 1H), 8.24 (d, *J* = 8.7 Hz, 1H).
MS (EI): m/z 466 (M+H).

### Example 22. Synthesis of Compound 22: (1-benzyl-6-methoxy-1H-indol-3-yl)-(3,4,5-trimethoxy-phenyl)-methanone

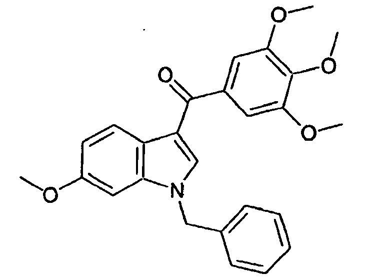

Compound 22 was prepared in a similar manner as described in Example 3.
¹H NMR (CDCl₃), A (ppm): 3.82 (s, 9H), 3.90 (s, 3H), 5.28 (s, 2H), 6.80 (d, *J*= 2.1 Hz, 1H), 6.98 (dd, *J* = 8.7, 2.1 Hz, 1H), 7.05 (s, 2H), 7.18 (m, 2H), 7.32 (m, 3H), 7.50 (s, 1H), 8.27 (d, *J* = 9.0 Hz, 1H).
MS (EI): m/z 432 (M+H).

### Example 23 Synthesis of Compound 23: (6-fluoro-1H-indol-3-yl)-(3,4,5-trimethoxy-phenyl)-methanone

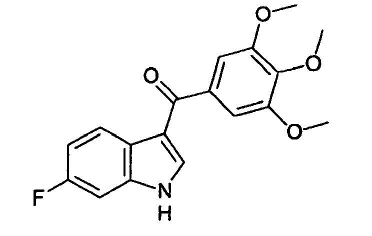

Compound 23 was prepared in a similar manner as described in Example 1.
¹H NMR (CDCl₃), A (ppm): 3.74 (s, 3H), 3.84 (s, 6H), 7.06-7.13 (m, 3H), 7.28 (dd, *J* = 9.6, 2.4 Hz, 1H), 8.10 (s, 1H), 8.19-8.23 (m, 1H), 12.06 (br, 1H,NH).
MS (EI): m/z 330 (M+H).

### Example 24. Synthesis of Compound 24: (6-bromo-1H-indol-3-yl)-(3,4,5-trimethoxy-phenyl)-methanone

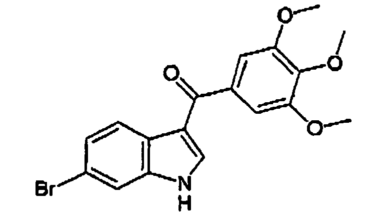

Compound 24 was prepared in a similar manner as described in Example 1.
¹H NMR (CDCl₃), A (ppm): 3.73 (s, 3H), 3.86 (s, 6H), 7.09 (s, 2H), 7.38 (dd, *J* = 8.4,1.8 Hz, 1H), 7.70 (d ,*J*= 1.2 Hz, 1H), 8.14 (s, 1H), 8.17 (d, *J* = 8.4 Hz, 1H), 12.13 (br, 1H,NH).
MS (EI): m/z 390 (M+H).

### Example 25. Synthesis of Compound 25: (4,5,6-trimethoxy-1H-indol-3-yl)-(3,4,5-trimethoxy-phenyl)-methanone

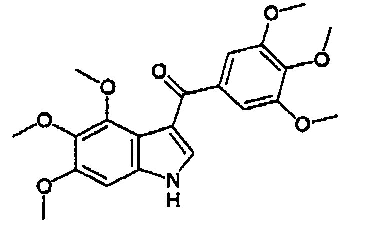

Compound 25 was prepared in a similar manner as described in Example 1.
¹H NMR (CDCl₃), A (ppm): 3.72 (s, 3H), 3.76 (s, 6H), 3.78 (s, 6H), 3.83 (s, 3H), 6.82 (s, 1H), 7.10 (s, 2H), 7.67 (d, *J* = 3.0 Hz, 1H), 11.69 (br, 1H, NH).
MS (EI): m/z 402 (M+H).

### Example 34. Synthesis of Compound 34: (5,6-dimethoxy-1H-indol-3-yl)-(3,4,5-trimethoxy-phenyl)-methanone

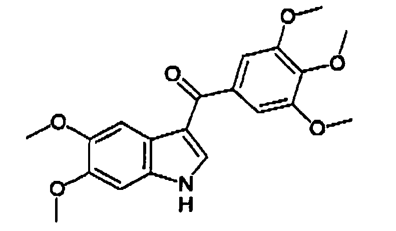

Compound 34 was prepared in a similar manner as described in Example 1.
¹H NMR (CDCl₃), A (ppm): 3.89 (s, 6H), 3.92 (s, 6H), 3.98 (s, 3H), 6.93 (s, 1H), 7.11 (s, 2H), 7.59 (d, *J*=2.7 Hz, 1H), 7.91 (s, 1H), 8.72 (s, 1H).
MS(EI): m/z 372 (M+H).

### Example 36. Synthesis of Compound 36: (1,6-dimethyl-1H-indol-3-yl)-(3,4,5-trimethoxy-phenyl)-methanone

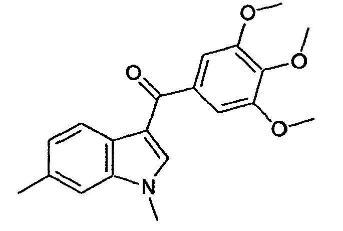

Compound 36 was prepared in a similar manner as described in Example 16.
¹H NMR (CDCl₃), A (ppm): 2.52 (s, 3H), 3.82 (2,3H), 3.89 (s, 6H), 3.93 (s, 3H), 7.08 (s, 2H), 7.18~7.15 (m, 2H), 7.52 (s, 1H), 8.23 (d, *J*=8.7 Hz, 1H).
MS(EI): m/z 340 (M+H).

### Example 37. Synthesis of Compound 37: (1-ethyl-6-methyl-1H-indol-3-yl)-(3,4,5-trimethoxy-phenyl)-methanone

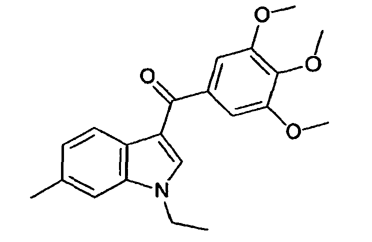

Compound 37 was prepared in a similar manner as described in Example 36.
¹H NMR (CDCl₃), A (ppm): 1.52 (t, *J*=7.3 Hz, 3H), 2.53 (s, 3H), 3.90 (s, 6H), 3.93 (s, 3H), 4.20 (q, *J* =7.3 Hz, 2H), 7.10 (s, 1H), 7.17 (d, *J* =8.4 Hz), 7.20 (s, 1H), 7.60 (s, 1 H), 8.23 (d, *J* =8.1 Hz, 1H).
MS(EI): m/z 354 (M+H).

### Example 38. Synthesis of Compound 38: (1-allyl-6-methyl-1H-indol-3-yl)-(3,4,5-trimethoxy-phenyl)-methanone

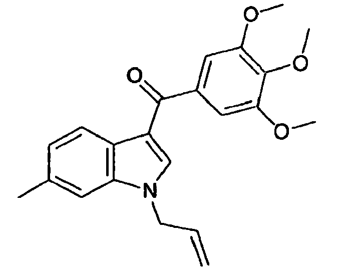

Compound 38 was prepared in a similar manner as described in Example 4.
¹H NMR (CDCl₃), Λ (ppm): 2.51 (s, 3H), 3.89 (s, 6H), 3.93 (s, 3H), 4.75 (dt, *J=*5.4, 1.5 Hz, 2H), 5.26 (dt, *J*=5.4, 1.5 Hz, 2H), 5.174 (dd, *J=17.1,* 0.9 Hz, 1H), 5.29 (dd, *J*=10.5, 1.2 Hz, 1H), 7.10 (s, 2H), 7.18~7.15 (m, 2H), 7.57 (s, 1H), 8.24 (d, *J*=8.4 Hz, 1H).
MS(EI): m/z 366 (M+H).

### Example 39. Synthesis of Compound 39: (5-ethyl-5H-[1.3]dioxolo[4,5-f]indol-7-yl)-(3,4,5-trimethoxy-phenyl)-methanone

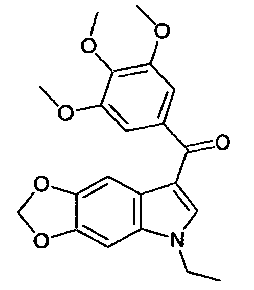

Compound 39 was prepared in a similar manner as described in Example 37.
¹H NMR (CDCl₃), A (ppm): 1.47 (t, *J*=7.3 Hz, 3H), 3.90 (s, 6H), 3.93 (s, 3H), 4.132 (q, *J*= 7.3Hz, 2H), 6.84 (s, 1H), 6.00 (s, 2H), 7.08 (s, 2H), 7.50 (s, 1H), 7.84 (s, 1H).
MS(EI): m/z 384 (M+H).

### Example 40. Synthesis of Compound 40: (5-methyl-5H-[1,3]dioxolo[4,5-f]indol-7-yl) - (3,4,5-trimethoxy-phenyl)-methanone

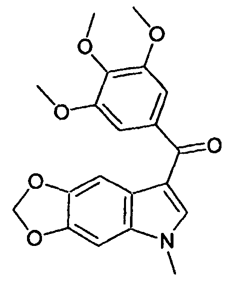

Compound 40 was prepared in a similar manner as described in Example 36.
¹H NMR (CDCl₃), Λ (ppm): 3.78 (s, 3H), 3.90 (s, 6H), 3.93 (s, 3H), 6.01 (s, 2H), 6.81 (d, *J*=0.5 Hz, 1H), 7.07 (s, 2H), 7.43 (s, 1H), 7.83 (d, *J*=1 Hz, 1H).
MS(EI): m/z 370 (M+H).

### Example 41. Synthesis of Compound 41: (5-allyl-5H-[1,3]dioxolo[4,5-f]indol-7-yl)- (3,4,5-trimethoxy-phenyl)-methanone

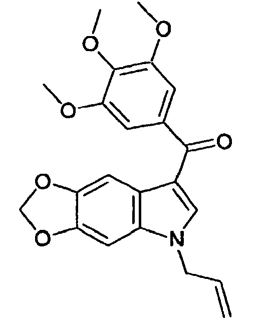

Compound 41 was prepared in a similar manner as described in Example 38.
¹H NMR (CDCl₃), Λ (ppm): 3.90 (s, 6H), 3.93 (s, 3H), 4.69 (dt, *J*=5.4, 1.5 Hz, 2H), 5.15 (dd, *J*=17, 0.9 Hz, 1H), 5.29 (dd, *J*=9, 0.9 Hz, 1H), 6.03-5.94 (m , 3H), 7.68 (d,*J*=1 Hz, 1H), 7.08 (s, 2H), 7.48 (s, 1H), 7.84 (d, *J*=1 Hz, 1H).
MS(EI): m/z 396 (M+H).

### Example 42. Synthesis of Compound 42: (6-methoxy-2-methyl-1H-indol-3-yl-(3,4,5-trimethoxy-phenyl)-methanone

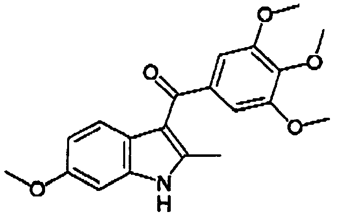

Compound 42 was prepared in a similar manner as described in Example 1.
¹H NMR (CDCl₃), Λ (ppm): 2.52 (s, 3H), 3.80 (s, 9H), 3.91 (s, 3H), 6.73 (dd, *J*=8.7, 2.1 Hz, 1H), 6.79 (d, *J*=2.4 Hz, 1H), 7.05 (s, 2H), 7.35 (d, *J*=8.7 Hz, 1H), 8.50 (s, 1H).
MS(EI): m/z 356 (M+H).

### Example 44. Synthesis of Compound 44: 6-ethoxy-1H-indol-3-yl)(3,4,5- trimethoxy-phenyl)-methanone

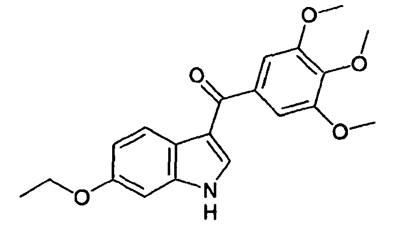

Compound 44 was prepared in a similar manner as described in Example 1.
¹H NMR (CDCl₃), Λ (ppm): 1.47 (t, *J*= 7 Hz, 3H), 3.9 (s, 6H), 3.92 (s, 3H), 4.09 (q, *J*= 6.9 Hz, 2H), 6.92 (d, *J*= 2.4 Hz, 1H), 6.99 (dd, *J =* 8.7, 2.1 Hz, 1H), 7.12(s, 2H), 7.63 (d, *J* = 2.7 Hz, 1H), 8.63 (br, 1H, NH).
MS(EI): m/z 356 (M+H).

### Example 45. Synthesis of Compound 45: (7-methoxy-1H-indol-3-yl-3,4,5- trimethoxy-phenyl)-methanone

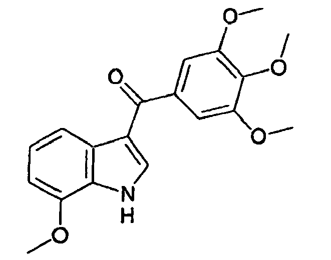

Compound 45 was prepared in a similar manner as described in Example 1.
¹H NMR (CDCl₃), Λ (ppm): 3.90(s, 6H), 3.94(s, 3H), 3.99(s, 3H), 6.78(d, *J*= 7.8 Hz, 2H), 7.13(s, 2H), 7.26 (d, *J*= 7.8 Hz, 1H), 7.71 (d, *J*=3 Hz, 1H), 7.93(d, *J=* 8.1, 1H), 8.96 (br, 1H, NH).
MS(EI): m/z 342 (M+H).

### Example 46. Synthesis of Compound 46: (4-Methoxy-1H-indol-3-yl)-(3,4,5-trimethoxy-phenyl)-methanone

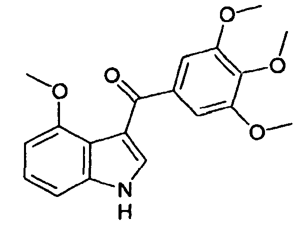

Compound 46 was prepared in a similar manner as described in Example 1.
¹H NMR (CDCl₃), Λ (ppm): 3.79(s, 6H), 3.85(s, 3H), 3.94(s, 3H), 6.66(d, *J*= 7.8 Hz, 1H), *7.08*(d*, J=*8.1 Hz, 1H), 7.19(s, 2H), 7.23(d, *J*=8.1 Hz, 1H), 7.54(d,*J*=8.1 Hz, 1H), 8.71 (br, 1H, NH).
MS(EI): m/z 342 (M+H).

### Example 47. Synthesis of Compound 47: (5-Methoxy-4-methyl-1H-indol-3-yl)-(3,4,5-trimethoxy-phenyl)-methanone

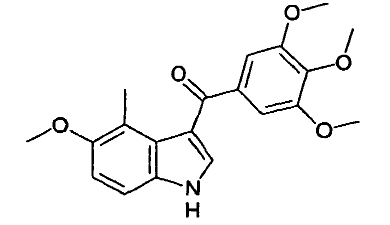

Compound 47 was prepared in a similar manner as described in Example 1.
¹H NMR (CDCl₃), Λ (ppm): 2.56 (s, 3H), 3.87 (s, 6H), 3.95 (s, 3H), 6.99 (d, *J*= 8.7 Hz, 1H), 7.19 (s, 2H), 7.22 (d, *J* = 9.3 Hz, 1H), 7.43 (d, *J* = 3 Hz, 1H), 9.10 (br, 1H, NH).
MS(EI): m/z 356 (M+H).

### Example 48. Synthesis of Compound 48: (4,7-dimethoxy-1H-indol-3-yl)-(3,4,5-trimethoxy-phenyl)-methanone

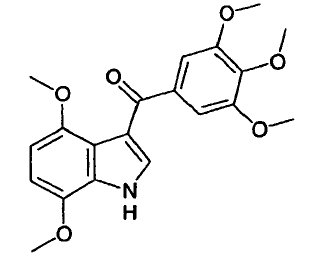

Compound 48 was prepared in a similar manner as described in Example 1.
¹H NMR (CDCl₃), Λ (ppm): 3.74 (s, 3H), 3.84 (s, 6H), 3.93 (s, 3H), 3.94 (s, 3H), 6.51 (d, *J* = 8.4, 1H), 6.64 (d, *J =* 8.4,1H), 7.17 (s, 2H), 7.53 (d, *J* = 2.7 Hz, 1H), 9.00 (br, 1H, NH).
MS(EI): m/z 372 (M+H).

### Example 49. Synthesis of Compound 49: (4.6-dimethoxy-1H-indol-3-yl)-(3,4,5-trimethoxy-phenyl)-methanone

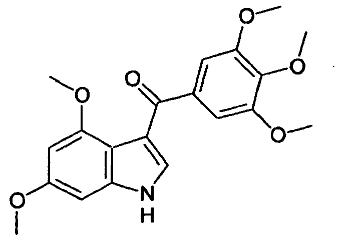

Compound 49 was prepared in a similar manner as described in Example 1.
¹H NMR (CDCl₃), Λ (ppm): 3.79 (s, 3H), 3.85 (s, 3H), 3.86(s, 6H), 3.94 (s, 3H), 6.33 (d, *J* = 2.1 Hz, 1H), 6.55 (d, *J* = 1.8 Hz, I H), 7.17(s, 2H), 7.45 (d, *J =* 2.7 Hz, 1H), 9.17 (br, I H, NH).
MS(EI): m/z 372 (M+H).

### Example 50. Synthesis of Compound 50: (5,7-dimethoxy-1H-indol-3-yl)-(3,4,5-trimethoxy-phenyl)-methanone

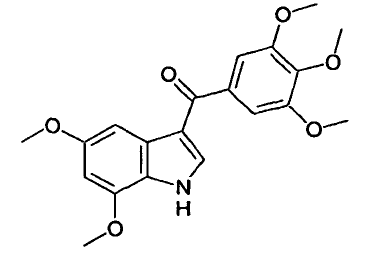

Compound 50 was prepared in a similar manner as described in Example 1.
¹H NMR (CDCl₃), Λ (ppm): 3.85~3.97 (m, 15H), 6.46 (d, *J*= 2.1 Hz, 1H), 7.48 (d, *J* = 2.1 Hz, 1H), 7.65 (d, *J*=3.3 Hz, 1H), 8.79 (br, 1H, NH).
MS(EI): m/z 372 (M+H).

### Example 51. Synthesis of Compound 51: {6-methoxy-1-[4-(4-nitro-phenyl)-furan-2-ylmethyl]-1H-indol-3-yl}-(3,4,5-trimethoxy-phenyl)-methanone

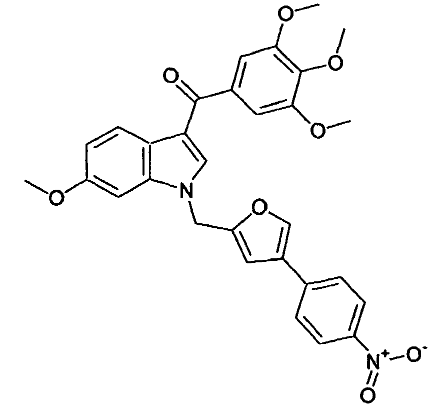

Compound 51 was prepared in a similar manner as described in Example 9.
¹H NMR (CDCl₃), Λ (ppm): 3.90 (s, 6H), 3.93 (s, 3H), 5.50 (s, 2H), 6.45 (s, 1H), 6.87 (d, *J*= 1.8 Hz, 1H), 7.01 (dd, *J =* 8.7, 2.1 Hz, 1H), 7.11 (s, 2H), 7.66 (s, 1H), 7.89 (dd, *J* = 6.6, 2.0 Hz, 1H), 8.27 (dd, *J*= 6.5, 2.0 Hz, 1H), 9.08 (br, I H, NH).
MS (EI): m/z 541 (M+H).

### Example 52. Synthesis of Compound 52: (6-hydroxy-1H-indol-3-yl)-(3,4,5- trimethoxy-phenyl)-methanone

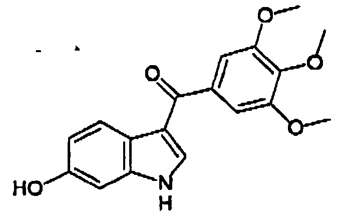

Compound 52 was prepared in a similar manner as described in Example 1.
¹H NMR (CDCl₃), Λ (ppm): 3.87 (s, 3H), 3.88 (s, 6H), 6.80 (dd, J = 8.4, 2.1 Hz, 1H), 6.86 (d, J = 2.1 Hz, 1H), 7.08 (s, 2H), 7.65 (d, J = 4.2 Hz, 1H), 8.03 (d, J = 8.7 Hz, 1H), 8.95 (br, 1H).
MS (EI): m/z 328 (M+H).

### Example 54. Synthesis of Compound 54: [1-(2-dimethylamino-ethyl)-4,5,6- trimethoxy-1H-indol- 3-yl]-(3,4,5-trimethoxy-phenyl)-methanone

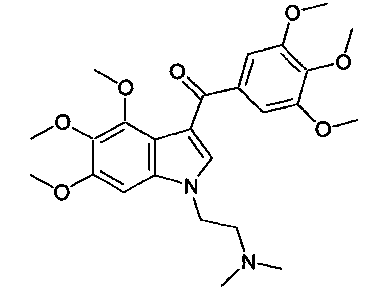

Compound 54 was prepared in a similar manner as described in Example 15.
¹H NMR (CDCl₃), Λ (ppm): 2.29(s, 6H), 2.70(t, J=6.8Hz, 2H), 3,87(s, 6H), 3.89(s, 3H), 3.92(s, 3H), 3.94(s, 3H), 3.94(s, 3H), 4.15(t, J=6.8Hz, 2H), 6.62(s, 1H), 7.15(s, 2H), 7.41 (s, 3H).
MS(EI): m/z 473 (M+H).

### Example 55. Synthesis of Compound 55: (6-propoxy-1H-indol-3-yl)-(3,4,5-trimethoxy-phenyl)-methanone

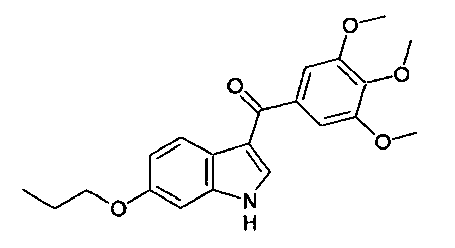

Compound 55 was prepared in a similar manner as described in Example 1.
¹H NMR (CDCl₃), δ (ppm): 1.03 (t, *J* =7.5 Hz, 3H), 1.81 (h, *J* = 7.2 Hz, 2H), 3.83 (s, 6H), 3.84 (m, 2H), 3.91 (m, 3H), 6.85 (d, *J* = 2.1 Hz, 1H), 6.94 (dd, *J* = 8.7, 2.4 Hz, 1H), 7.07 (s, 2H), 7.57 (d, *J* = 3 Hz, 1H), 8.21 (d, *J* = 8.7 Hz, 1H), 9.61 (br, 1H, NH).
MS (EI): m/z 370 (M+H).

### Example 56. Synthesis of Compound 56: (6-isopropoxy-1H-indol-3-yl)-(3,4,5-trimethoxy-phenyl)-methanone

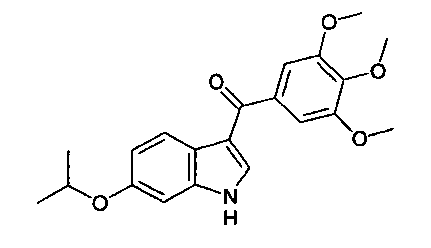

Compound 56 was prepared in a similar manner as described in Example 1.
¹H NMR (CDCl₃), δ (ppm): 1.28 (d, *J*=6 Hz, 6H), 3.80 (s, 6H), 3.93 (s, 3H), 4.48 (m, 1H), 6.87 (d, *J* = 2.1 Hz, 1H), 6.92 (dd, *J* = 8.7, 2.1 Hz, 1H), 7.07 (s, 2H), 7.59 (d, *J* = 3 Hz, 1H), 8.21 (d, *J* = 8.7 Hz, 1H), 9.75 (br, 1H, NH).
MS (El): m/z 370 (M+H).

### Example 57 Synthesis of Compound 57: (3 5-dimethoxy-phenyl)-(6-methoxy-1H-indol-3-yl)-methanone

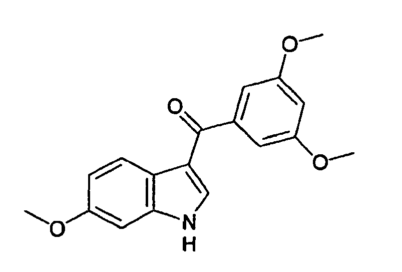

Compound 57 was prepared in a similar manner as described in Example 1.
¹H NMR (CDCl₃), δ (ppm): ¹H NMR: 3.84 (s, 6H), 3.87 (s, 3H), 6.64(t, *J*= 2.1 Hz, 1H), 6.91 (d, *J* = 2.4 Hz, 1H), 6.97 (d, *J* = 2.4 Hz, 2H), 7.01 (m, 1H), 7.63 (d, *J* = 2.7 Hz,, I H), 8.31 (d,*J*=9.0 Hz, 1H), 8.76 (br, 1H, NH).
MS (EI): m/z 312 (M+H).

### Example 58. Synthesis of Compound 58: (3,4-dimethoxy-phenyl)-(6-methoxy-1H-indol-3-yl)-methanone

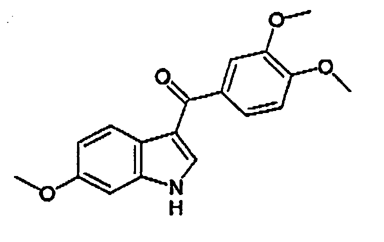

Compound 58 was prepared in a similar manner as described in Example 1.
¹H NMR (CDCl₃), δ (ppm): 3.88 (s, 3H), 3.97 (s, 3H), 3.98 (s, 3H), 6.81(d, *J*= 2.1 Hz, 1H), 6.84 (d, *J*= 2.1 Hz, 1H), 6.86(d, *J*= 2.1 Hz, 1H), 6.96(d, *J*= 8.4 Hz, 1H), 7.10 (dd, *J*=2.1, 0.9 Hz,, 1H), 7.52(d, *J*= 2.1 Hz, 1H), 7.57 (d, *J*= 8.7 Hz, 1H), 7.68 (dd, *J*= 8.4, 2.1 Hz,, 1H), 9.25 (br, 1H, NH).
MS (EI): m/z 312 (M+H).

### Example 60. Synthesis of Compound 60: [6-(3-morpholin-4-yl-propoxy)-1H-indol-3-yl]-(3,4,5-trimethoxy-phenyl)-methanone

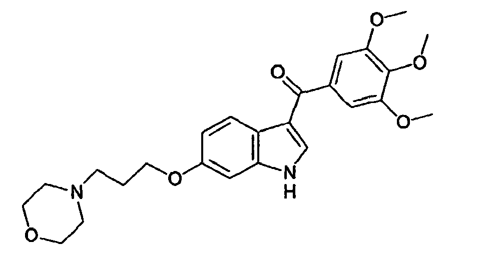

Compound 60 was prepared in a similar manner as described in Example 1.
¹H NMR (CDCl₃), δ (ppm): 2.02 (m, 2H), 2.49-2.60 (m, 6H), 2.02 (m, 2H), 3.74 (m, 4H), 3.89 (s, 6H), 3.91 (s, 3H), 4.06 (t, *J*= 6.3 Hz, 2H), 6.89 (d, *J*= 2.1 Hz, 1H), 6.96 (dd, *J* =8.7, 2.4 Hz, 1H), 7.09 (s, 2H), 7.61 (d, *J* = 3.0 Hz, 1H), 8.22 (d, *J* = 8.7Hz, 1H), 8.77 (br, 1H,NH).
MS (EI): m/z 455 (M+H).

### Example 61. Synthesis of Compound 61: (6- {2-[2-(2-hydroxy-ethoxy)-ethoxy]-ethoxy}-1H-indol-3-yl)-(3,4,5-trimethoxy-phenyl)-methanone

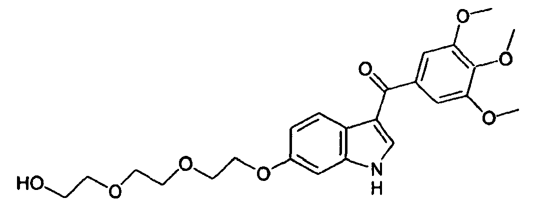

Compound 61 was prepared in a similar manner as described in Example 1.
¹H NMR (CDCl₃), δ (ppm): 3.54-3.80 (m, 8H), 3.88-3.91 (m, 11H), 4.17-4.20 (m., 2H), 6.93 (dd, J = 8.7, 2.4 Hz, 1H), 7.04 (d, J = 2.1 Hz, 1H), 7.10 (s, 2H), 7.80 (s, 1H), 8.11 (d, J = 8.4 Hz, 1H).
MS (EI): m/z 460 (M+H).

### Example 62. In vitro assays

*Cell Growth Inhibition Assay:* MCF-7 breast carcinoma cells were maintained in plastic dishes in DMEM medium supplemented with 10% fetal bovine serum. For *in vitro* treatment, tumor cells were seeded in 100 mL of culture medium/well in 96-well plates to a final cell density of 6 x 10³ cell/mL and incubated in a CO₂ incubator at 37 °C for 24 h. The cells were treated with at least five different concentrations of a test indole compound, and incubated in a CO₂ incubator at 37 °C for 72 h. The number of viable cells was estimated using MTS assay and absorbance was measured at 490 nm. Cytotoxicity of the test compounds was expressed in terms of IC₅₀ values. The values presented represent averages of three independent experiments, each with duplicate samples.

In addition, indole compounds described in the "Summary" section were also test on HT-29 colon carcinoma cell line, as well as Hepa-G2 hepatic cell line. At least 28 compounds have IC₅₀ values of at least 5 µM. Unexpectedly, some of the test compounds have IC₅₀ values as low as <10 nM.

*Tubulin Polymerization Assay:* Turbidimetric assays of microtubule were performed as described by Lopes et al. (1997, Cancer Chemother. Pharmacol. 41: 37-47) and manual of Cytoskeleton with some modification. MAP-rich tubulin (2 mg/ml) was preincubated in polymerization buffer (0.1 M PIPES, pH 6.9, 1 mM MgCl₂) with drug at 4°C for 2 min before the addition of 1 mM GTP. The samples were then rapidly warmed to 37 °C in a 96-well plate thermostatically controlled spectrophotometer and measuring the change at 350 nm with time. Results show that a test indole compound of 2 µM inhibited tubulin polymerization.

### Cell Growth Inhibition Assay on Multiple-drug Resistant Human Cancer Lines:

Indole compounds were tested against several panels of drug-resistant cell lines. It is well known that several anti-mitotic agents, including vinca alkaloid (vincristine, vinblastine) and taxol, have been introduced in clinic to treat various human cancers. Vinca alkaloid resistance has been attributed to a number of mechanisms associated with multi-drug resistance (MDR) phenotype including overexpression of p-glycoprotein and the multi-drug resistant-associated protein (MRP). The mechanisms responsible for taxol resistance include overexpression of p-glycoprotein and mutation of tubulin. For comparison, three anti-mitotic agents, i.e., Vincristine, VP-16, Cisplatin, CPT (camptothecin), and Taxol (paclitaxel) were also tested against several panels of drug-resistant cell lines.

KB-Vin10, a vincristine-resistant cell line derived from its parental cell line KB, showed over expression of p-glycoprotein. HONECis-6, derived from cell line HONE-1, showed resistant to an alkylating agent such as cisplatin. The mechanism of the cisplatin-resistance is under investigation. KB 100, i.e., camptotnecin (CPT)-resistant cell line, displayed down regulation of topoisomerase I and an undefined mechanism responsible for drug resistance. The mechanisms responsible for VP16-resistance (KB7D) were down-regulation of topoisomerase II and overexpression of MRP 1. CPT30, a CPT-resistant cell line showed quantitatively and qualitative change of topoisomerase I. KBtaxol-5 showed mutation of tubulin.

In addition, indole compounds described in the "Summary" section were also tested on the A549 lung cell line, MESSA uterine cell line, MKN45 stomach cell line, SW480 colon cell line, NUGC3 stomach cell line, and MESSA/DX5 uterine cell line.

The results shown in Tables 1 and 2 indicate that indole compounds described in the "Summary" section are strong anti-mitotic agents, and are useful in treatment of various cancers, including multiple-drug resistant cancers.

**Table 1. Cytotoxicity of indole compounds against various human cancer resistant cell lines (nM)**

| Cell line | KB | KB -Vin10 | HONE -1 | HONECis -6 | KB100 | KB100R | KB7D | KB7DR | KBtaxol -5 |
|---|---|---|---|---|---|---|---|---|---|
| Cell type | Oral | Vin. Res | NPC | Cis Res. | CPT Res | Reverse CPT Res | VP-16 Res. | Reverse VP-16 Res. | Taxol Res. |
| Compound 1 | 6 | 7 | 4 | 4.5 | 8 | 12 | 4 | 6 | 7 |
| Compound 6 | 13 | 9.6 | 19 | 19 | 18 | 18 | 8 | 15 | 37 |
| Compound 27 | 94 | 85 | 40 | 40 | 83 | 81 | 41 | 46 | 100 |
| Vincristine | 1 | 43 | 0.5 | 0.6 | | | 1920 | 337 | |
| VP-16 | 500 | | | | | | 44000 | 3500 | |
| Cisplatin | 3 | 5 | 3000 | 15000 | | | | | |
| CPT | 26 | 400 | | | 1286 | | 133 | | |
| Taxol | 10 | | | | | | | | 80 |

**Table 2. Cytotoxicity of indole compounds against various human cancer cell lines (nM)**

| Cell line | A549 | MESSA | MKN45 | SW480 | NUGC | MCF7 | MESSA/DX5 | HONE-1 |
|---|---|---|---|---|---|---|---|---|
| Cell type | Lung | Uterine | Stomach | Colon | Stomach | Breast | Uterine | NPC |
| Compound 1 | 17.0 | 7.0 | 4.0 | 3.0 | 3.0 | 7.6 | 3.8 | 4.0 |
| Compound 33 | 6.0 | 3.7 | 3.0 | 3.3 | 3.6 | 0.9 | 2.7 | 2.5 |

### Example 63. In vivo assay

*CAM Assay for Antiangiogenic Potency:* Test compounds were dissolved in a 2.5% aqueous agarose solution (final concentration: 1-20 mg/mL). For the preparation of the pellets, 10 µL of these solutions were applied dropwise on circular Teflon supports of 3 mm in diameter and then cooled to room temperature at once. After incubation at 37 °C and relative humidity of 80% for 65-70 h, the fertilized hens' eggs were positioned in a horizontal position and rotated several times. Before the opening on the snub side, 10 mL of albumin were aspirated from a hole on the pointed side. At two-third of the height (from the pointed side), the eggs were traced with a scalpel, and the shells were removed with forceps. The aperture (cavity) was covered with keep-fresh film, and the eggs were incubated at 37 °C at a relative humidity of 80% for 75 h. When the formed chorioallantoic membrane (CAM) had approximately a diameter of 2 cm, one pellet (1 pellet/ egg) was placed on it. The eggs were incubated for 1 day and subsequently evaluated under the stereomicroscope. Three compounds were tested, and all show anti-angiogenesis activities.

*Tumor Regression Models:* The procedures of cancer cell implantation were similar to those previously reported in Chen et al., Cancer Res., (1998) 58:2777-2783, with modifications. Male CD 1 nude mice (5~6 weeks old) were purchased from Charles River Laboratories (Wilmington, MA, USA). Human MKN45 and KB cancer cells were maintained in RPMI 1640 plus 10% fetal bovine serum. Each animal was subcutaneously implanted with 5×10⁵ MKN45 or 1×10⁶ KB cells in 0.1 ml at one flank. Tumor growth was examined three times a week after implantation. The volume of tumor was measured with a caliper and calculated as 1/2×length×width² in mm³. Test compounds were dissolved completely in a vehicle mixture of dimethyl sulfoxide:cremophor:saline (1:4:15). When the size of a growing tumor reached approximately 100 mm³, the mice were treated with test compounds intravenously from tail veins at a concentration of 50 mg/kg daily for two weeks. Mice in a control group were treated with the solvent vehicle only. Tumor sizes and body weights were measured twice a week and after the mice were sacrificed.

Compound I and cisplatin were tested. When tested on cell line MKN45, Compound I exhibited higher activity in inhibiting tumor growth than cisplatin. When tested on cell lines KB and MKN 45, Compound 1 and cisplatin administered together exhibited higher activity in inhibiting tumor growth than cisplatin administered alone.

## Claims

1. A compound of the following formula: 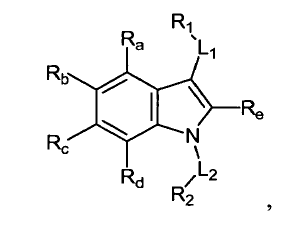 wherein
L₁ isC(O);
L₂ is a bond;
R₁ is 5, 6, or 7-membered aryl or heteroaryl tri-substituted with alkyloxy;
R₂ is H, aryl, heteroaryl, halogen, nitro, nitroso, cyano, azide, isothionitro, OR, OC(O)R, OC(O)OR, OC(O)NRR', SO₂R SO₃R, SO₂NRR', SR, NRR', NRSO₂NR'R" NRSO₂R', NRSO₃R', NRC(O)R', NRC(O)NR'R", NRC(O)OR', NRC(N)NR'R", C(O)OR, C(O)NRR', an amino acid moiety, a polypeptide moiety, a carbohydrate moiety, or a PEG moiety, in which each of said aryl or heteroaryl, independently is unsubstituted or optionally substituted with halogen, cyano, nitro, hydroxyl, amino, mercapto, alkyl, alkenyl, alkynyl, aryl, heteroaryl, cyclyl, heterocyclyl, alkyloxy, aryloxy, alksulfanyl, arylsulfanyl, alkylamino, arylamino, dialkylamino, diarylamino, alkylcarbonyl, arylcarbonyl, heteroarylcarbonyl, alkylcarboxyl, arylcarboxyl, heteroarylcarboxyl, alkyloxycarbonyl, aryloxycarbonyl, heteroaryloxycarbonyl, alkylcarbamido, arylcarbamido, heterocarbamido, alkylcarbamyl, arylcarbamyl, heterocarbamyl;
each of Rₐ, R_{b}, R_{c}, and R_{d}, independently, is R, halogen, nitro, nitroso, cyano, azide, isothionitro, OR, OC(O)R, OC(O)OR, OC(O)NRR', SO₂R SO₃R, SO₂NRR', SR, NRR', NRSO₂NR'R" NRSO₂R' NRSO₃R', NRC(O)R', NRC(O)NR'R", NRC(O)OR', NRC(N)NR'R", C(O)R, C(O)OR, C(O)NRR', OP(O)(OR)(OR'), O(CH₂)ₙOP(O)(OR)(OR'), or O(CH₂)ₙ-PEG, or R_{b} and R_{c}, Rₐ and R_{b}, or R_{c} and R_{d} taken together are O(CH₂)ₙO; and
Rₑ is H or C₁-C₆ alkyl, in which said C₁-C₆ alkyl is unsubstituted or optionally substituted with halogen, cyano, nitro, hydroxyl, amino, mercapto, alkyl, alkenyl, alkynyl, cyclyl, heterocyclyl, alkyloxy, alksulfanyl, alkylamino, dialkylamino, alkylcarbonyl, alkylcarboxyl, alkyloxycarbonyl, alkylcarbamido, heterocarbamido, alkylcarbamyl or heterocarbamyl;
in which each of R, R', and R", independently, is H, C₁-C₆ alkyl, C₂-C₆ alkenyl, C₂-C₆ alkynyl, aryl, heteroaryl, cyclyl, or heterocyclyl;
n is 1,2,3,4, or 5; and
in which each of said C₁-C₆ alkyl, C₂-C₆ alkenyl, C₂-C₆ alkynyl, aryl, heteroaryl, cyclyl, or heterocyclyl, independently is unsubstituted or optionally substituted with halogen, cyano, nitro, hydroxyl, amino, mercapto, alkyl, alkenyl, alkynyl, aryl, heteroaryl, cyclyl, heterocyclyl, alkyloxy, aryloxy, alksulfanyl, arylsulfanyl, alkylamino, arylamino, dialkylamino, diarylamino, alkylcarbonyl, arylcarbonyl, heteroarylcarbonyl, alkylcarboxyl, arylcarboxyl, heteroarylcarboxyl, alkyloxycarbonyl, aryloxycarbonyl, heteroaryloxycarbonyl, alkylcarbamido, arylcarbamido, heterocarbamido, alkylcarbamyl, arylcarbamyl, heterocarbamyl.

2. The compound of claim 1, wherein R₁ is 3,4,5-trimethoxylphenyl.

3. The compound of claim 1, wherein Rₑ is H.

4. The compound of claim 3, wherein R₁ is 3,4,5-trimethoxylphenyl.

5. The compound of claim 4, wherein each of Ra, R_{b}, R_{c}, and R_{d}, independently, is H, alkyloxy, C₁-C₆ alkyl, or halogen.

6. The compound of claim 5, wherein R_{c} is alkyloxy, C₁-C₆ alkyl, or halogen, and each of Rₐ, R_{b}, and R_{d} is H.

7. The compound of claim 6, wherein R₂ is H, OR, SO₂R, C(O)OR, or C(O)NRR', in which each of R and R', independently, is H, C₁-C₆ alkyl, aryl, or heteroaryl.

8. The compound of claim 4, wherein R_{b} and R_{c} taken together are O(CH₂)ₙO, and each of Rₐ and R_{d} is H, in which n is 1 or 2.

9. The compound of claim 1, wherein the compound is
(6-methoxy-1H-indol-3-yl)-(3,4,5-trimethoxy-phenyl)-methanone;
(6-methyl-1H-indol-3-yl)-(3,4,5-trimethoxy-phenyl)-methanone;
6-methoxy-3-(3,4,5-trimethoxy-benzoyl)-indole-1-carboxylic acid tert-butyl ester;
(1-methanesulfonyl-6-methoxy-1H-indol-3-yl)-(3,4,5-trimethoxy-phenyl)-methanone;
6-methoxy-3-(3,4,5-trimethoxy-benzoyl)-indole-1-carboxylic acid dimethylamide;
6-methoxy-3-(3,4,5-trimethoxy-benzoyl)-indole-1-carboxylic acid phenyl ester;
[1-(5-dimethylamino-naphthalene-1-sulfonyl)-6-methoxy-1H-indol-3-yl]-(3,4,5-trimethoxy-phenyl)-methanone;
(6-fluoro-1H-indol-3-yl)-(3,4,5-trimethoxy-phenyl)-methanone;
(6-bromo-1H-indol-3-yl)-(3,4,5-trimethoxy-phenyl)-methanone;
(4,5,6-trimethoxy-1H-indol-3-yl)-(3,4,5-trimethoxy-phenyl)-methanone;
(5,6-dimethoxy-1H-indol-3-yl)-(3,4,5- trimethoxy-phenyl)-methanone;
(6-methoxy-2-methyl-1H-indol-3-yl)-(3,4,5-trimethoxy-phenyl)-methanone;
(6-ethoxy-1H-indol-3-yl)-(3,4,5-trimethoxy-phenyl)-methanone;
(7-methoxy-1H-indol-3-yl)-(3,4,5-trimethoxy-phenyl)-methanone;
(4-methoxy-1H-indol-3-yl)-(3,4,5-trimethoxy-phenyl)-methanone;
(5-methoxy-4-methyl-1H-indol-3-yl)-(3,4,5-trimethoxy-phenyl)-methanone;
(4,7-dimethoxy-1H-indol-3-yl)-(3,4,5- trimethoxy-phenyl)-methanone;
(4,6-dimethoxy-1H-indol-3-yl)-(3,4,5-trimethoxy-phenyl)-methanone;
(5,7-dimethoxy-1H-indol-3-yl)-(3,4,5-trimethoxy-phenyl)-methanone;
(6-hydroxy-1H-indol-3-yl)-(3,4,5- trimethoxy-phenyl)-methanone;
(6-propoxy-1H-indol-3-yl)-(3,4,5-trimethoxy-phenyl)-methanone;
(6-isopropoxy-1H-indol-3-yl)-(3,4,5-trimethoxy-phenyl)-methanone;
(6-methoxy-1H-indol-3-yl)-phenyl-methanone;
[6-(3-morpholin-4-yl-propoxy)-1H-indol-3-yl]-(3,4,5-trimethoxy-phenyl)-methanone;
(6-{2-[2-(2-hydroxy-ethoxy)-ethoxy]-ethoxy}-1H-indol-3-yl)-(3,4,5-trimethoxy-phenyl)-methanone;
4-[6-methoxy-3-(3,4,5-trimethoxy-benzoyl)-indole-1-sulfonyl]-benzoic acid; or
(5H-[1,3]dioxolo[4,5-f]indol-7-yl)-(3,4,5-trimethoxy-phenyl)-methanone.

10. The compound of claim 1, wherein the compound is (6-methoxy-1H-indol-3-yl)-(3,4,5-trimethoxy-phenyl)-methanone.

11. A compound of the following formula: 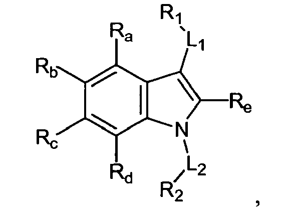 wherein
L₁ is C(O);
L₂ is a bond;
R₁ is 5, 6, or 7-membered aryl or heteroaryl tri-substituted with alkyloxy;
R₂ is C₁-C₆ alkyl, C₂-C₆ alkenyl, C₂-C₆ alkynyl, cyclyl, or heterocyclyl, in which each of said C₁-C₆ alkyl, C₂-C₆ alkenyl, C₂-C₆ alkynyl, cyclyl, or heterocyclyl, independently is unsubstituted or optionally substituted with halogen, cyano, nitro, hydroxyl, amino, mercapto, alkyl, alkenyl, alkynyl, aryl, heteroaryl, cyclyl, heterocyclyl, alkyloxy, aryloxy, alksulfanyl, arylsulfanyl, alkylamino, arylamino, dialkylamino, diarylamino, alkylcarbonyl, arylcarbonyl, heteroarylcarbonyl, alkylcarboxyl, arylcarboxyl, heteroarylcarboxyl, alkyloxycarbonyl, aryloxycarbonyl, heteroaryloxycarbonyl, alkylcarbamido, arylcarbamido, heterocarbamido, alkylcarbamyl, arylcarbamyl, heterocarbamyl;
each of Rₐ, R_{b}, R_{c}, and R_{d}, independently, is R, halogen, nitro, isothionitro, OR, OC(O)R, OC(O)OR, OC(O)NRR', SO₂R SO₃R, SO₂NRR', SR, NRSO₂NR'R" NRSO₂R' NRSO₃R', NRC(O)R', NRC(O)NR'R", NRC(O)OR', NRC(N)NR'R", C(O)R, C(O)OR, C(O)NRR', OP(O)(OR)(OR'), O(CH₂)ₙOP(O)(OR)(OR'), or O(CH₂)ₙ-PEG, or R_{b} and R_{c}, Rₐ and R_{b}, or R_{c}, and R_{d} taken together are O(CH₂)ₙO; and
Rₑ is H or C₁-C₆ alkyl, in which said C₁-C₆ alkyl, is unsubstituted or optionally substituted with halogen, cyano, nitro, hydroxyl, amino, mercapto, alkyl, alkenyl, alkynyl, cyclyl, heterocyclyl, alkyloxy, alksulfanyl,alkylamino, dialkylamino, alkylcarbonyl, alkylcarboxyl, alkyloxycarbonyl, alkylcarbamido, heterocarbamido, alkylcarbamyl or heterocarbamyl;
in which each ofR, R', and R", independently, is H, C₁-C₆ alkyl, C₂-C₆ alkenyl, C₂-C₆ alkynyl, aryl, heteroaryl, cyclyl, or heterocyclyl;
n is 1, 2, 3, 4, or 5; and
in which each of said C₁-C₆ alkyl, C₂-C₆ alkenyl, C₂-C₆ alkynyl, aryl, heteroaryl, cyclyl, or heterocyclyl, independently is unsubstituted or optionally substituted with halogen, cyano, nitro, hydroxyl, amino, mercapto, alkyl, alkenyl, alkynyl, aryl, heteroaryl, cyclyl, heterocyclyl, alkyloxy, aryloxy, alksulfanyl, arylsulfanyl, alkylamino, arylamino, dialkylamino, diarylamino, alkylcarbonyl, arylcarbonyl, heteroarylcarbonyl, alkylcarboxyl, arylcarboxyl, heteroarylcarboxyl, alkyloxycarbonyl, aryloxycarbonyl, heteroaryloxycarbonyl, alkylcarbamido, arylcarbamido, heterocarbamido, alkylcarbamyl, arylcarbamyl, heterocarbamyl.

12. The compound of claim 11, wherein R₁ is 3,4,5-trimethoxylphenyl.

13. The compound of claim 11, wherein Rₑ is H.

14. The compound of claim 13, wherein R₁ is 3,4,5-trimethoxylphenyl.

15. The compound of claim 14, wherein each of Rₐ, R_{b}, R_{c}, and R_{d}, independently, is H, alkyloxy, C₁-C₆ alkyl, or halogen.

16. The compound of claim 15, wherein R_{c} is alkyloxy, C₁-C₆ alkyl, or halogen, and each of Rₐ, R_{b}, and R_{d} is H.

17. The compound of claim 16, wherein R₂ is C₁-C₆ alkyl, C₂-C₆ alkenyl, or C₂-C₆ alkynyl. 1.

18. The compound of claim 14, wherein R_{b} and R_{c} taken together are O(CH₂)ₙO, and each of Rₐ and R_{d} is H, in which n is 1 or 2.

19. The compound of claim 11, wherein the compound is
(6-methoxy-1-pyridin-4-ylmethyl-1H-indol-3-yl)-(3,4,5-trimethoxy-phenyl)-methanone;
(1-allyl-6-methoxy-1H-indol-3-yl)-(3,4,5-trimethoxy-phenyl)-methanone;
[6-methoxy-1-(2-piperidin-1-yl-ethyl)-1H-indol-3-yl]-(3,4,5-trimethoxy-phenyl)-methanone;
(6-methoxy-1-prop-2-ynyl-1H-indol-3-yl)-(3,4,5-trimethoxy-phenyl)-methanone;
[1-(2-dimethylamino-ethyl)-6-methoxy-1H-indol-3-yl]-(3,4,5-trimethoxy-phenyl)-methanone;
(6-methoxy-1-methyl-1H-indol-3-yl)-(3,4,5-trimethoxy-phenyl)-methanone;
[1-(2-amino-ethyl)-6-methoxy-1H-indol-3-yl]-(3,4,5-trimethoxy-phenyl)-methanone;
(1-ethyl-6-methoxy-1H-indol-3-yl)-(3,4,5-trimethoxy-phenyl)-methanone;
[6-methoxy-1-(2-morpholin-4-yl-ethyl)-1H-indol-3-yl]-(3,4,5-trimethoxy-phenyl)-methanone;
[1-(4-chloro-benzyl)-6-methoxy-1H-indol-3-yl]-(3,4,5-trimethoxy-phenyl)-methanone;
(1-benzyl-6-methoxy-1H-indol-3-yl)-(3,4,5-trimethoxy-phenyl)-methanone;
(1,6-dimethyl-1H-indol-3-yl)-(3,4,5- trimethoxy-phenyl)-methanone;
(1-ethyl-6-methyl-1H-indol-3-yl)-(3,4,5- trimethoxy-phenyl)-methanone;
(1-allyl-6-methyl-1H-indol-3-yl)-(3,4,5-trimethoxy-phenyl)-methanone;
(5-ethyl-5H-[1,3]dioxolo[4,5-f]indol-7-yl)-(3,4,5-trimethoxy-phenyl)-methanone;
(5-methyl-5H-[1,3]dioxolo[4,5-f]indol-7-yl)-(3,4,5-trimethoxy-phenyl)-methanone;
(5-allyl-5H-[1,3]dioxolo[4,5-f]indol-7-yl)-(3,4,5-trimethoxy-phenyl)-methanone;
{6-methoxy-1-[4-(4-nitro-phenyl)-furan-2- ylmethyl]-1H-indol-3-yl}-(3,4,5-trimethoxy-phenyl)-methanone; or
[1-(2-dimethylamino-ethyl)-4,5,6-trimethoxy-1H-indol-3-yl]-(3,4,5-trimethoxy-phenyl)-methanone.

20. A compound of the following formula: 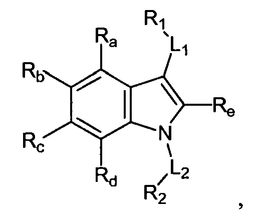 wherein
L₁ is C(O);
L₂ is a bond;
R₁ is 5, 6, or 7-membered aryl or heteroaryl tri-substituted with alkyloxy;
R₂ is COR"';
each of Rₐ, R_{b}, R_{c}, and R_{d}, independently, is R, halogen, nitro, nitroso, cyano, azide,
isothionitro, OR, OC(O)R, OC(O)OR, OC(O)NRR', SO₂R SO₃R, SO₂NRR', SR, NRR', NRSO₂NR'R", NRSO₂R', NRSO₃R', NRC(O)R', NRC(O)NR'R", NRC(O)OR', NRC(N)NR'R", C(O)R, C(O)OR, C(O)NRR', OP(O)(OR)(OR'), O(CH₂)ₙOP(O)(OR)(OR'), or O(CH₂)ₙ-PEG, or R_{b} and R_{c}, Rₐ and R_{b}, or R_{c} and R_{d} taken together are O(CH₂)ₙO; and
Rₑ is H or C₁-C₆ alkyl, in which said C₁-C₆ alkyl, is unsubstituted or optionally substituted with halogen, cyano, nitro, hydroxyl, amino, mercapto, alkyl, alkenyl, alkynyl, cyclyl, heterocyclyl, alkyloxy, alksulfanyl,alkylamino, dialkylamino, alkylcarbonyl, alkylcarboxyl, alkyloxycarbonyl, alkylcarbamido, heterocarbamido, alkylcarbamyl, heterocarbamyl;
in which each of R, R', and R" independently, is H, C₁-C₆ alkyl, C₂-C₆ alkenyl, C₂-C₆ alkynyl, aryl, heteroaryl, cyclyl, or heterocyclyl; R"' is H, C₁-C₆ alkyl, C₂-C₆ alkenyl, C₂-C₆ alkynyl, heteroaryl, cyclyl, or heterocyclyl;
n is 1, 2, 3, 4, or 5; and
in which each of said C₁-C₆ alkyl, C₂-C₆ alkenyl, C₂-C₆ alkynyl, aryl, heteroaryl, cyclyl, or heterocyclyl, independently is unsubstituted or optionally substituted with halogen, cyano, nitro, hydroxyl, amino, mercapto, alkyl, alkenyl, alkynyl, aryl, heteroaryl, cyclyl, heterocyclyl, alkyloxy, aryloxy, alksulfanyl, arylsulfanyl, alkylamino, arylamino, dialkylamino, diarylamino, alkylcarbonyl, arylcarbonyl, heteroarylcarbonyl, alkylcarboxyl, alkylcarbamido, arylcarbamido, heterocarbamido, alkylcarbamyl, arylcarbamyl, heterocarbamyl.

21. The compound of claim 20, wherein R₁ is 3,4,5-trimethoxylphenyl.

22. The compound of claim 20, wherein Rₑ is H.

23. The compound of claim 22, wherein R₁ is 3,4,5-trimethoxylphenyl.

24. The compound of claim 23, wherein each of Rₐ, R_{b}, R_{c}, and R_{d}, independently, is H, alkyloxy, C₁-C₆ alkyl, or halogen.

25. The compound of claim 24, wherein R_{c} is alkyloxy, C₁-C₆ alkyl, or halogen, and each of Rₐ, R_{b}, and R_{d} is H.

26. The compound of claim 25, wherein R"' is C₁-C₆ alkyl, C₂-C₆ alkenyl, heterocyclyl, or heteroaryl.

27. The compound of claim 20, wherein the compound is
[6-methoxy-1-(pyridine-2-carbonyl)-1H-indol-3-yl]-(3,4,5-trimethoxy-phenyl)-methanone;
[6-methoxy-1-(morpholine-4-carbonyl)-1H-indol-3-yl]-(3,4,5-trimethoxy-phenyl)-methanone;
1-[6-methoxy-3-(3,4,5-trimethoxy-benzoyl)-indol-1-yl]-3-phenyl-propenone;
[1-(furan-2-carbonyl)-6-methoxy-1H-indol-3-yl]-(3,4,5-trimethoxy-phenyl)-methanone;
{2-[6-methoxy-3-(3,4,5-trimethoxy-benzoyl)-indol-1-yl]-2-oxo-ethyl}-carbamic acid 9H-fluoren-9-yl-methyl ester;
[6-methoxy-1-(pyridine-3-carbonyl)-1H-indol-3-yl]-(3,4,5-trimethoxy-phenyl)-methanone; or
[6-methoxy-1-(thiophene-2-carbonyl)-1H-indol-3-yl]-(3,4,5-trimethoxy-phenyl)-methanone.

28. A compound which is one of:
(3,5-dimethoxy-phenyl)-(6-methoxy-1H-indol-3-yl)-methanone; and
(3,4-dimethoxy-phenyl)-(6-methoxy-1H-indol-3-yl)-methanone.

29. A compound of claim 1, 11, 20 or 28 for use in the treatment of cancer.

30. A compound of claim 1, 11, 20 and 28 for use in the inhibition of tubulin polymerization in a subject in need thereof.

31. A compound of claim 1, 11, 20 or 28 for use in the treatment of an angiogenesis-related disorder.

32. A compound of claim 9, 10, 19, or 27 for use in the manufacture of the medicament for the treatment of cancer.

## Patentansprüche

1. Eine Verbindung mit der folgenden Formel: 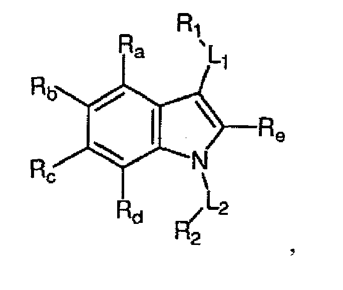 wobei
L₁ C(O) ist;
L₂ eine Bindung ist;
R₁ mit Alkyloxy trisubstituiertes 5-, 6- oder 7-gliedriges Aryl oder Heteroaryl ist;
R₂ H, Aryl, Heteroaryl, Halogen, Nitro, Nitroso, Cyano, Azid, Isothionitro, OR, OC(O)R, OC(O)OR, OC(O)NRR', SO₂R, SO₃R, SO₂NRR', SR, NRR', NRSO₂NR'R", NRSO₂R', NRSO₃R', NRC(O)R', NRC(O)NR'R", NRC(O)OR', NRC(N)NR'R", C(O)OR, C(O)NRR', eine Aminosäuregruppe, eine Polypeptidgruppe, eine Kohlenhydratgruppe oder eine PEG-Gruppe ist, in dem jedes von besagtem Aryl oder Heteroaryl unabhängig unsubstituiert oder optional substituiert ist mit Halogen, Cyano, Nitro, Hydroxyl, Amino, Mercapto, Alkyl, Alkenyl, Alkinyl, Aryl, Heteroaryl, Cyclyl, Heterocyclyl, Alkyloxy, Aryloxy, Alkylsulfanyl, Arylsulfanyl, Alkylamino, Arylamino, Dialkylamino, Diarylamino, Alkylcarbonyl, Arylcarbonyl, Heteroarylcarbonyl, Alkylcarboxyl, Arylcarboxyl, Heteroarylcarboxyl, Alkyloxycarbonyl, Aryloxycarbonyl, Heteroaryloxycarbonyl, Alkylcarbamido, Arylcarbamido, Heterocarbamido, Alkylcarbamyl, Arylcarbamyl, Heterocarbamyl.;
jedes von Rₐ, R_{b}, Rₑ und R_{d} unabhängig R, Halogen, Nitro, Nitroso, Cyano, Azid, Isothionitro, OR, OC(O)R, OC(O)OR, OC(O)NRR', SO₂R, SO₃R, SO₂NRR', SR, NRR', NRSO₂NR'R", NRSO₂R', NRSO₃R', NRC(O)R', NRC(O)NR'R", NRC(O)OR', NRC(N)NR'R", C(O)R, C(O)OR, C(O)NRR', OP(O)(OR)(OR'), O(CH₂)ₙOP(O)(OR)(OR') oder O(CH₂)ₙ-PEG ist oder R_{b} und R_{c}, Rₐ und R_{b}, oder Rₑ und R_{d} zusammengenommen O(CH₂)ₙO sind; und
Rₑ H oder C₁-C₆-Alkyl ist, wobei besagtes C₁-C₆-Alkyl unsubstituiert oder optional substituiert ist mit Halogen, Cyano, Nitro, Hydroxyl, Amino, Mercapto, Alkyl, Alkenyl, Alkinyl, Cyclyl, Heterocyclyl, Alkyloxy, Alkylsulfanyl, Alkylamino, Dialkylamino, Alkylcarbonyl, Alkylcarboxyl, Alkyloxycarbonyl, Alkylcarbamido, Heterocarbamido, Alkylcarbamyl oder Heterocarbamyl;
wobei jedes von R, R' und R" unabhängig H, C₁-C₆-Alkyl, C₂-C₆-Alkenyl, C₂-C₆-Alkinyl, Aryl, Heteroaryl, Cyclyl oder Heterocyclyl ist;
n 1, 2, 3, 4 oder 5 ist; und
wobei jedes von besagtem C₁-C₆-Alkyl, C₂-C₆-Alkenyl, C₂-C₆-Alkinyl, Aryl, Heteroaryl, Cyclyl oder Heterocyclyl unabhängig unsubstituiert oder optional substituiert ist mit Halogen, Cyano, Nitro, Hydroxyl, Amino, Mercapto, Alkyl, Alkenyl, Alkinyl, Aryl, Heteroaryl, Cyclyl, Heterocyclyl, Alkyloxy, Aryloxy, Alkylsulfanyl, Arylsulfanyl, Alkylamino, Arylamino, Dialkylamino, Diarylamino, Alkylcarbonyl, Arylcarbonyl, Heteroarylcarbonyl, Alkylcarboxyl, Arylcarboxyl, Heteroarylcarboxyl, Alkyloxycarbonyl, Aryloxycarbonyl, Heteroaryloxycarbonyl, Alkylcarbamido, Arylcarbamido, Heterocarbamido, Alkylcarbamyl, Arylcarbamyl, Heterocarbamyl.

2. Die Verbindung aus Anspruch 1, wobei R₁ 3,4,5-Trimethoxyphenyl ist.

3. Die Verbindung aus Anspruch 1, wobei RₑH ist.

4. Die Verbindung aus Anspruch 3, wobei R₁ 3,4,5-Trimethoxyphenyl ist.

5. Die Verbindung aus Anspruch 4, wobei jedes von Rₐ, R_{b}, R_{c} und R_{d} unabhängig H, Alkyloxy, C₁-C₆-Alkyl oder Halogen ist.

6. Die Verbindung aus Anspruch 5, wobei R_{c} Alkyloxy, C₁-C₆-Alkyl oder Halogen ist und jedes von Rₐ, R_{b} und R_{d} H ist.

7. Die Verbindung aus Anspruch 6, wobei R₂ H, OR, SO₂R, C(O)OR oder C(O)NRR' ist, wobei jedes von R und R' unabhängig H, C₁-C₆-Alkyl, Aryl oder Heteroaryl ist.

8. Die Verbindung aus Anspruch 4, wobei R_{b} und R_{c} zusammengenommen O(CH₂)ₙO sind und jedes von Rₐ und Rₐ H ist, wobei n 1 oder 2 ist.

9. Die Verbindung aus Anspruch 1, wobei die Verbindung
(6-Methoxy-1H-indol-3-yl)-(3,4,5-trimethoxyphenyl)-methanon;
(6-Methyl-1H-indol-3-yl)-(3,4,5-trimethoxyphenyl)-methanon;
6-Methoxy-3-(3,4,5-trimethoxybenzoyl)-indol-1-carbonsäure-tert.-butylester;
(1-Methansulfonyl-6-methoxy-1H-indol-3-yl)-(3,4,5-trimethoxyphenyl)-methanon;
6-Methoxy-3-(3,4,5-trimethoxybenzoyl)-indol-1-carbonsäure-dimethylamid;
6-Methoxy-3-(3,4,5-trimethoxybenzoyl)-indol-1-carbonsäure-phenylester;
[1-(5-Dimethylaminonaphthalin-1-sulfonyl)-6-methoxy-1H-indol-3-yl]- (3,4,5-trimethoxyphenyl)-methanon;
(6-fluor-1H-indol-3-yl)-(3,4,5-trimethoxyphenyl)-methanon;
(6-Brom-1H-indol-3-yl)-(3,4,5-trimethoxyphenyl)-methanon;
(4,5,6-Trimethoxy-1H-indol-3-yl)-(3,4,5-trimethoxyphenyl)-methanon;
(5,6-Dimethoxy-1H-indol-3-yl)-(3,4,5-trimethoxyphenyl)-methanon;
(6-Methoxy-2-methyl-1H-indol-3-yl)-(3,4,5-trimethoxyphenyl)-methanon;
(6-Ethoxy-1H-indol-3-yl)-(3,4,5-trimethoxyphenyl)-methanon;
(7-Methoxy-1H-indol-3-yl)-(3,4,5-trimethoxyphenyl)-methanon;
(4-Methoxy-1H-indol-3-yl)-(3,4,5-trimethoxyphenyl)-methanon;
(5-Methoxy-4-methyl-1H-indol-3-yl)-(3,4,5-trimethoxyphenyl)-methanon;
(4,7-Dimethoxy-1H-indol-3-yl)-(3,4,5-trimethoxyphenyl)-methanon;
(4,5-Dimethoxy-1H-indol-3-yl)-(3,4,5-trimethoxyphenyl)-methanon;
(5,7 -Dimethoxy-1H-indol-3-yl)-(3,4,5-trimethoxyphenyl)-methanon;
(6-Hydroxy-1H-indol-3-yl)-(3,4,5-trimethoxyphenyl)-methanon;
(6-Propoxy-1H-indol-3-yl)-(3,4,5-trimethoxyphenyl)-methanon;
(6-Isopropoxy-1H-indol-3-yl)-(3,4,5-trimethoxyphenyl)-methanon;
(6-Methoxy-1H-indol-3-yl)-phenylmethanon;
[6-(3-Morpholin-4-yl-propoxy)-1H-indol-3-yl]-(3,4,5-trimethoxyphenyl)-methanon;
(6-{2-[2-(2-Hydroxyethoxy)-ehtoxy]-ethoxy}-1H-indol-3-yl)-(3,4,5-trimethoxyphenyl)-methanon;
4-[6-Methoxy-3-(3,4,5-trimethoxybenzoyl)-indol-1-sulfonyl]-benzoesäure; oder
(5H-[1,3]Dioxolo[4,5-f]indol-7-yl)-(3,4,5-trimethoxyphenyl)-methanon ist.

10. Die Verbindung aus Anspruch 1, wobei die Verbindung (6-Methoxy-1H-indol-3-yl)-(3,4,5-trimethoxyphenyl)-methanon ist.

11. Eine Verbindung mit der folgenden Formel: 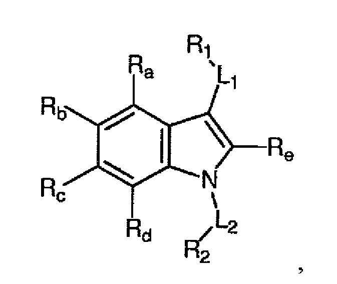 wobei
L₁ C(O) ist;
L₂ eine Bindung ist;
R₁ mit Alkyloxy trisubstituiertes 5-, 6- oder 7-gliedriges Aryl oder Heteroaryl ist;
R₂ C₁-C₆-Alkyl, C₂-C₆-Alkenyl, C₂-C₆-Alkinyl, Cyclyl oder Heterocyclyl ist, wobei jedes von besagtem C₁-C₆-Alkyl, C₂-C₆-Alkenyl, C₂-C₆-Alkinyl, Cyclyl oder Heterocyclyl unabhängig unsubstituiert oder optional substituiert ist mit Halogen, Cyano, Nitro, Hydroxyl, Amino, Mercapto, Alkyl, Alkenyl, Alkinyl, Aryl, Heteroaryl, Cyclyl, Heterocyclyl, Alkyloxy, Aryloxy, Alkylsulfanyl, Arylsulfanyl, Alkylamino, Arylamino, Dialkylamino, Diarylamino, Alkylcarbonyl, Arylcarbonyl, Heteroarylcarbonyl, Alkylcarboxyl, Arylcarboxyl, Heteroarylcarboxyl, Alkyloxycarbonyl, Aryloxycarbonyl, Heteroaryloxycarbonyl, Alkylcarbamido, Arylcarbamido, Heterocarbamido, Alkylcarbamyl, Arylcarbamyl, Heterocarbamyl;
jedes von Rₐ, R_{b}, R_{c} und R_{d} unabhängig R, Halogen, Nitro, Isothionitro, OR, OC(O)R, OC(O)OR, OC(O)NRR', SO₂R, SO₃R, SO₂NRR', SR, NRSO₂NR'R", NRSO₂R', NRSO₃R', NRC(O)R', NRC(O)NR'R", NRC(O)OR', NRC(N)NR'R", C(O)R, C(O)OR, C(O)NRR', OP(O)(OR)(OR'), O(CH₂)_{N}OP(O)(OR)(OR') oder O(CH₂)ₙ-PEG ist oder R_{b} und R_{c}, Rₐ und R_{b}, oder R_{c} und R_{d} zusammengenommen O(CH₂)ₙO sind; und
Rₑ H oder C₁-C₆-Alkyl ist, wobei besagtes C₁-C₆-Alkyl unsubstituiert oder optional substituiert ist mit Halogen, Cyano, Nitro, Hydroxyl, Amino, Mercapto, Alkyl, Alkenyl, Alkinyl, Cyclyl, Heterocyclyl, Alkyloxy, Alkylsulfanyl, Alkylamino, Dialkylamino, Alkylcarbonyl, Alkylcarboxyl, Alkyloxycarbonyl, Alkylcarbamido, Heterocarbamido, Alkylcarbamyl oder Heterocarbamyl;
wobei jedes von R, R' und R" unabhängig H, C₁-C₆-Alkyl, C₂-C₆-Alkenyl, C₂-C₆-Alkinyl, Aryl, Heteroaryl, Cyclyl oder Heterocyclyl ist;
n 1, 2, 3, 4 oder 5 ist; und
wobei jedes von besagtem C₁-C₆-Alkyl, C₂-C₆-Alkenyl, C₂-C₆-Alkinyl, Aryl, Heteroaryl, Cyclyl oder Heterocyclyl unabhängig unsubstituiert oder optional substituiert ist mit Halogen, Cyano, Nitro, Hydroxyl, Amino, Mercapto, Alkyl, Alkenyl, Alkinyl, Aryl, Heteroaryl, Cyclyl, Heterocyclyl, Alkyloxy, Aryloxy, Alkylsulfanyl, Arylsulfanyl, Alkylamino, Arylamino, Dialkylamino, Diarylamino, Alkylcarbonyl, Arylcarbonyl, Heteroarylcarbonyl, Alkylcarboxyl, Arylcarboxyl, Heteroarylcarboxyl, Alkyloxycarbonyl, Aryloxycarbonyl, Heteroaryloxycarbonyl, Alkylcarbamido, Arylcarbamido, Heterocarbamido, Alkylcarbamyl, Arylcarbamyl, Heterocarbamyl.

12. Die Verbindung aus Anspruch 11, wobei R₁ 3,4,5-Trimethoxyphenyl ist.

13. Die Verbindung aus Anspruch 11, wobei Rₑ H ist.

14. Die Verbindung aus Anspruch 13, wobei R₁ 3,4,5-Trimethoxyphenyl ist.

15. Die Verbindung aus Anspruch 14, wobei jedes von Rₐ, R_{b}, R_{c} und R_{d} unabhängig H, Alkyloxy, C₁-C₆-Alkyl oder Halogen ist.

16. Die Verbindung aus Anspruch 15, wobei R_{c} Alkyloxy, C₁-C₆-Alkyl oder Halogen ist und jedes von Rₐ, R_{b} und R_{d} H ist.

17. Die Verbindung aus Anspruch 16, wobei R₂ C₁-C₆-Alkyl, C₂-C₆-Alkenyl oder C₂-C₆-Alkinyl ist.

18. Die Verbindung aus Anspruch 14, wobei R_{b} und R_{c} zusammengenommen O(CH₂)ₙO sind und jedes von Rₐ und R_{d} H ist, wobei n 1 oder 2 ist.

19. Die Verbindung aus Anspruch 11, wobei die Verbindung
(6-Methoxy-1-pyridin-4-yl-methyl-1H-indol-3-yl)-(3,4,5-trimethoxyphenyl)-methanon;
(1-Allyl-6-methoxy-1H-indol-3-yl)-(3,4,5-trimethoxyphenyl)-methanon;
[6-Methoxy-1-(2-piperidin-1-yl-ethyl)-1H-indol-3-yl]-(3,4,5-trimethoxyphenyl)-methanon;
(6-Methoxy-1-prop-2-inyl-1H-indol-3-yl)-(3,4,5-trimethoxyphenyl)-methanon;
[1-(2-Dimethylaminoethyl)-6-methoxy-1H-indol-3-yl)-(3,4,5-trimethoxyphenyl)-methanon;
(6-Methoxy-1-methyl-1H-indol-3-yl)-(3,4,5-trimethoxyphenyl)-methanon;
[1-(2-Aminoethyl)-6-methoxy-1H-indol-3-yl)-(3,4,5-trimethoxyphenyl)-methanon;
(1-Ethyl-6-methoxy-1H-indol-3-yl)-(3,4,5-trimethoxyphenyl)-methanon;
[6-Methoxy-1-(2-morpholin-4-yl-ethyl)-1H-indol-3-yl]-(3,4,5-trimethoxyphenyl)-methanon;
[1-(4-Chlorbenzyl)-6-methoxy-1H-indol-3-yl]-(3,4,5-trimethoxyphenyl)-methanan;
(1-Benzyl,6-methoxy-1H-indol-3-yl)-(3,4,5-trimethoxyphenyl)-methanon;
(1,6-Dimethyl-1H-indol-3-yl)-(3,4,5-trimethoxyphenyl)-methanon;
(1-Ethyl-6-methyl-1H-indol-3-yl)-(3,4,5-trimethoxyphenyl)-methanon;
(1-Allyl-6-methyl-1H-indol-3-yl)-(3,4,5-trimethoxyphenyl)-methanon;
(5-Ethyl-5H-[1,3]dioxolo[4,5-f]indol-7-yl)-(3,4,5-trimethoxyphenyl)-methanon;
(5-Methyl-5H-[1,3]dioxolo[4,5-f]indol-7-yl)-(3,4,5-trimethoxyphenyl)-methanon;
(5-Allyl-5H-[1,3]dioxolo[4,5-f]indol-7-yl)-(3,4,5-trimethoxyphenyl)-methanon;
{6-Methoxy-1-[4-(4-nitrophenyl)-furan-2-yl-methyl]-1H-indol-3-yl}-(3,4,5-trimethoxyphenyl)-methanon; oder
[1-(2-Dimethylaminoethyl)-4,5,6-trimethoxy-1H-indol-3-yl]-(3,4,5-trimethoxyphenyl)-methanon ist.

20. Eine Verbindung mit der folgenden Formel: 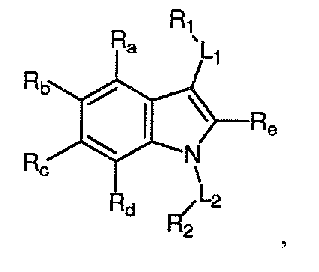 wobei
L₁ C(O) ist;
L₂ eine Bindung ist;
R₁ mit Alkyloxy trisubstituiertes 5-, 6- oder 7-gliedriges Aryl oder Heteroaryl ist; R₂ COR"' ist;
jedes von Rₐ, R_{b}, R_{c} und R_{d} unabhängig R, Halogen, Nitro, Nitroso, Cyano, Azid, Isothionitro, OR, OC(O)R, OC(O)OR, OC(O)NRR', SO₂R, SO₃R, SO₂NRR', SR, NRR', NRSO₂NR'R", NRSO₂R', NRSO₃R', NRC(O)R', NRC(O)NR'R", NRC(O)OR', NRC(N)NR'R", C(O)R, C(O)OR, C(O)NRR', OP(O)(OR)(OR'), O(CH₂)ₙOP(O)(OR)(OR') oder O(CH₂)ₙ-PEG ist oder R_{b} und R_{c}, Rₐ und R_{b}, oder R_{c} und R_{d} zusammengenommen O(CH₂)ₙO sind; und
Rₑ H oder C₁-C₆-Alkyl ist, wobei besagtes C₁-C₆-Alkyl unsubstituiert oder optional substituiert ist mit Halogen, Cyano, Nitro, Hydroxyl, Amino, Mercapto, Alkyl, Alkenyl, Alkinyl, Cyclyl, Heterocyclyl, Alkyloxy, Alkylsulfanyl, Alkylamino, Dialkylamino, Alkylcarbonyl, Alkylcarboxyl, Alkyloxycarbonyl, Alkylcarbamido, Heterocarbamido, Alkylcarbamyl oder Heterocarbamyl;
wobei jedes von R, R' und R" unabhängig H, C₁-C₆-Alkyl, C₂-C₆-Alkenyl, C₂-C₆-Alkinyl, Aryl, Heteroaryl, Cyclyl oder Heterocyclyl ist; R"' H, C₁-C₆-Alkyl, C₂-C₆-Alkenyl, C₂-C₆-Alkinyl, Heteroaryl, Cyclyl oder Heterocyclyl ist;
n 1, 2, 3, 4 oder 5 ist; und
wobei jedes von besagtem C₁-C₆-Alkyl, C₂-C₆-Alkenyl, C₂-C₆-Alkinyl, Aryl, Heteroaryl, Cyclyl oder Heterocyclyl unabhängig unsubstituiert oder optional substituiert ist mit Halogen, Cyano, Nitro, Hydroxyl, Amino, Mercapto, Alkyl, Alkenyl, Alkinyl, Aryl, Heteroaryl, Cyclyl, Heterocyclyl, Alkyloxy, Aryloxy, Alkylsulfanyl, Arylsulfanyl, Alkylamino, Arylamino, Dialkylamino, Diarylamino, Alkylcarbonyl, Arylcarbonyl, Heteroarylcarbonyl, Alkylcarboxyl, Arylcarboxyl, Heteroarylcarboxyl, Alkyloxycarbonyl, Aryloxycarbonyl, Heteroaryloxycarbonyl, Alkylcarbamido, Arylcarbamido, Heterocarbamido, Alkylcarbamyl, Arylcarbamyl, Heterocarbamyl.

21. Die Verbindung aus Anspruch 20, wobei R₁ 3,4,5-Trimethoxyphenyl ist.

22. Die Verbindung aus Anspruch 20, wobei Rₑ H ist.

23. Die Verbindung aus Anspruch 22, wobei R₁ 3,4,5-Trimethoxyphenyl ist.

24. Die Verbindung aus Anspruch 23, wobei jedes von Rₐ, R_{b}, R_{c} und R_{d} unabhängig H, Alkyloxy, C₁-C₆-Alkyl oder Halogen ist.

25. Die Verbindung aus Anspruch 24, wobei R_{c} Alkyloxy, C₁-C₆-Alkyl oder Halogen ist und jedes von Rₐ, R_{b} und R_{d} H ist.

26. Die Verbindung aus Anspruch 25. wobei R"' C₁-C₆-Alkyl, C₂-C₆-Alkenyl, Heterocyclyl oder Heteroaryl ist.

27. Die Verbindung aus Anspruch 20, wobei die Verbindung
[6-Methoxy-1-(pyridin-2-carbonyl)-1H-indol-3-yl)-(3,4,5-trimethoxyphenyl)-methanon;
[6-Methoxy-1-(morpholin-4-carbonyl)-1H-indol-3-yl)-(3,4,5-trimethoxyphenyl)-methanon;
1-[6-Methoxy-3-(3,4,5-trimethoxybenzoyl)-indol-1-yl]-3-phenylpropenon;
[1-(Furan-2-carbonyl)-5-methoxy-1H-indol-3-yl)-(3,4,5-trimethoxyphenyl)-methanon;
{2-[6-Methoxy-3-(3,4,5-trimethoxybenzoyl)-indol-1-yl]-2-oxoethyl}-carbamidsäure-9H-fluoren-9-yl-methylester;
[6-Methoxy-1-(pyridine-3-carbonyl)-1H-indol-3-yl)-(3,4,5-trimethoxyphenyl)-methanon; oder
[6-Methoxy-1-(thiophen-2-carbonyl)-1H-indol-3-yl)-(3,4,5-trimethoxyphenyl)-methanon ist.

28. Eine Verbindung, die eine von
(3,5-Dimethoxyphenyl)-(6-methoxy-1H-indol-3-yl)-methanon; und
(3,4-Dimethoxyphenyl)-(6-methoxy-1H-indol-3-yl)-methanon ist.

29. Eine Verbindung aus Anspruch 1, 11, 20 oder 28 zur Verwendung in der Behandlung von Krebs.

30. Eine Verbindung aus Anspruch 1, 11, 20 oder 28 zur Verwendung in der Hemmung der Tubulinpolymerisation bei einem Subjekt, das diese benötigt.

31. Eine Verbindung aus Anspruch 1, 11, 20 oder 28 zur Verwendung in der Behandlung einer Angiogenese-verwandten Störung.

32. Eine Verbindung aus Anspruch 9, 10, 19 oder 27 zur Verwendung in der Herstellung eines Medikamentes zur Behandlung von Krebs.

## Revendications

1. Composé de la formule suivante : 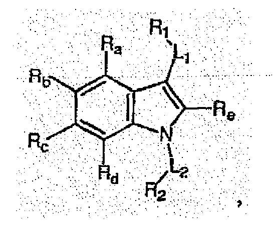 dans laquelle :
- L₁ représente C(O) ;
- L₂ représente une liaison ;
- R₁ représente aryle ou hétéroaryle à 5, 6 ou 7 chaînons trisubstitué par alkyloxy ;
- R₂ représente H, aryle, hétéroaryle, halogène, nitro, nitroso, cyano, azide, isothionitro, OR, OC(O)R, OC(O)OR, OC(O)NRR', SO₂R, SO₃R, SO₂NRR' , SR, NRR', NRSO₂NR'R", NRSO₂R' , NRSO₃R', NRC(O)R', NRC(O)NR'R'' , NRC(O)OR' , NRC(N)NR'R'', C(O)OR, C(O)NRR', une faction acide aminé, une fraction polypeptide, une fraction glucide ou une fraction PEG, où chacun dudit aryle ou hétéroaryle est indépendamment non substitué ou facultativement substitué par halogène, cyano, nitro, hydroxyle, amino, mercapto, alkyle, alcényle, alcynyle, aryle, hétéroaryle, cyclyle, hétérocyclyle, alkyloxy, aryloxy, alkylsulfanyle, arylsulfanyle, alkylamino, arylamino, dialkylamino, diarylamino, alkylcarbonyle, arylcarbonyle, hétéroarylcarbonyle, alkylcarboxyle, arylcarboxyle, hétéroarylcarboxyle, alkyloxycarbonyle, aryloxycarbonyle, hétéroaryloxycarbonyle, alkylcarbamido, arylcarbamido, hétérocarbamido, alkylcarbamyle, arylcarbamyle, hétérocarbamyle ;
- Rₐ, R_{b}, R_{c} et R_{d} représentent chacun indépendamment R, halogène, nitro, nitroso, cyano, azide, isothionitro, OR, OC(O)R, OC(O)OR, OC(O)NRR' , SO₂R, SO₃R, SO₂NRR', SR, NRR', NRSO₂NR'R", NRSO₂R', NRSO₃R', NRC(O)R', NRC(O)NR'R", NRC(O)OR', NRC(N)NR'R", C(O)R, C(O)OR, C(O)NRR', OP(O) (OR) (OR'), O(CH₂)ₙOP(O) (OR) (OR') ou O (CH₂)ₙ-PEG, ou R_{b} et R_{c}, Rₐ et R_{b}, ou R_{c} et R_{d} pris conjointement représentent O(CH₂)ₙO ; et
- Rₑ représente H ou alkyle en C₁-C₆, où ledit alkyle en C₁-C₆ est non substitué ou facultativement substitué par halogène, cyano, nitro, hydroxyle, amino, mercapto, alkyle, alcényle, alcynyle, cyclyle, hétérocyclyle, alkyloxy, alkylsulfanyle, alkylamino,, dialkylamino, alkylcarbonyle, alkylcarboxyle, alkyloxycarbonyle, alkylcarbamido, hétérocarbamido, alkylcarbamyle ou hétérocarbamyle ;
où R, R' et R" représentent indépendamment H, alkyle en C₁-C₆, alcényle en C₂-C₆, alcynyle en C₂-C₆, aryle, hétéroaryle, cyclyle ou hétérocyclyle ;
n vaut 1, 2, 3, 4 ou 5 ; et
où chacun dudit alkyle en C₁-C₆, alcényle en C₂-C₆, alcynyle en C₂-C₆, aryle, hétéroaryle, cyclyle ou hétérocyclyle est indépendamment non substitué ou facultativement substitué par halogène, cyano, nitro, hydroxyle, amino, mercapto, alkyle, alcényle, alcynyle, aryle, hétéroaryle cyclyle, hétérocyclyle, alkyloxy, aryloxy, alkylsulfanyle, arylsulfanyle, alkylamino, arylamino, dialkylamino, diarylamino, alkylcarbonyle, arylcarbonyle, hétéroarylcarbonyle, alkylcarboxyle, arylcarboxyle, hétéroarylcarboxyle, alkyloxycarbonyle, aryloxycarbonyle, hétéroaryloxycarbonyle, alkylcarbamido, arylcarbamido, hétérocarbamido, alkylcarbamyl, arylcarbamyle, hétérocarbamyle.

2. Composé selon la revendication 1, dans lequel R₁ représente 3,4,5-triméthoxyphényle.

3. Composé selon la revendication 1, dans lequel Rₑ représente H.

4. Composé selon la revendication 3, dans lequel R₁ représente 3,4,5-triméthoxylphényle.

5. Composé selon la revendication 4, dans lequel Rₐ, R_{b}, R_{c} et R_{d} représentent chacun indépendamment H, alkyloxy, alkyl en C₁-C₆ ou halogène.

6. Composé selon la revendication 5, dans lequel Rₑ représente alkyloxy, alkyle en C₁-C₆ ou halogène, et Rₐ, R_{b} et R_{d} représentent chacun H.

7. Composé selon la revendication 6, dans lequel R₂ représente H, OR, SO₂R, C(O)OR ou C(O)NRR', dans lequel R et R' représentent chacun indépendamment H, alkyle en C₁-C₆, aryle ou hétéroaryle.

8. Composé selon la revendication 4, dans lequel R_{b} et Rₑ pris conjointement représentent O(CH₂)ₙO, et Rₐ et R_{d} représentent chacun H, où n vaut 1 ou 2.

9. Composé selon la revendication 1, dans lequel le composé est :
- la (6-méthoxy-1H-indol-3-yl)-(3,4,5-triméthoxy-phényl)-méthanone ;
- la (6-méthyl-1H-indol-3-yl)-(3,4,5-triméthoxy-phényl)-méthanone ;
- l'ester tert-butylique de l'acide 6-méthoxy-3-(3,4,5-triméthoxy-benzoyl)-indole-1-carboxylique ;
- la (1-méthanesulfonyl-6-méthoxy-1H-indol-3-yl)-(3,4,5-triméthoxy-phényl)-méthanone ;
- le diméthylamide de l'acide 6-méthoxy-3-(3,4,5-triméthoxy-benzoyl)-indole-1-carboxylique ;
- l'ester phénylique de l'acide 6-méthoxy-3-(3,4,5-triméthoxy-benzoyl)-indole-1-carbaxylique ;
- la [1-(5-diméthylamino-naphtalène-1-sulfonyl)-6-méthoxy-1H-indol-3-yl]-(3,4,5-triméthoxy-phényl)-méthanone ;
- la (6-fluoro-1H-indol-3-yl)-(3,4,5-triméthoxy-phényl)-méthanone ;
- la (6-bromo-1H-indol-3-yl)-(3,4,5-triméthoxy-phényl)-méthanone ;
- la (4,5,6-triméthoxy-1H-indol-3-yl)-(3,4,5-triméthoxy-phényl)-méthanone ;
- la (5,6-diméthoxy-1H-indol-3-yl)-(3,4,5-triméthoxy-phényl)-méthanone ;
- la (6-méthoxy-2-méthyl-1H-indol-3-yl)-(3,4,5-triméthoxy-phényl)-méthanone ;
- la (6-éthoxy-1H-indol-3-yl)-(3,4,5-triméthoxy-phényl)-méthanone ;
- la (7-méthoxy-1H-indol-3-yl)-(3,4,5-triméthoxy-phényl)-méthanone ;
- la (4-méthoxy-1H-indol-3-yl)-(3,4,5-triméthoxy-phényl)-méthanone ;
- la (5-méthoxy-4-méthyl-1H-indol-3-yl)-(3,4,5-triméthoxy-phényl)-méthanone ;
- la (4,7-diméthoxy-1H-indol-3-yl)-(3,4,5-triméthoxy-phényl)-méthanone ;
- la (4,6-diméthoxy-1H-indol-3-yl)-(3,4,5-triméthoxy-phényl)-méthanone ;
- la (5,7-diméthoxy-1H-indol-3-yl)-(3,4,5-triméthoxy-phényl)-méthanone ;
- la (6-hydroxy-1H-indol-3-yl)-(3,4,5-triméthoxy-phényl)-méthanone ;
- la (6-propoxy-1H-indol-3-yl)-(3,4,5-triméthoxy-phényl)-méthanone ;
- la (6-isopropoxy-1H-indol-3-yl)-(3,4,5-triméthoxy-phényl)-méthanone ;
- la (6-méthoxy-1H-indol-3-yl)-phényl-méthanone ;
- la [6-(3-morpholin-4-yl-propoxy)-1H-indol-3-yl]-(3,4,5-triméthoxy-phényl)-méthanone ;
- la (6-{2-[2-(2-hydroxy-éthoxy)-éthoxy]-éthoxy}-1H-indol-3-yl)-(3,4,5-triméthoxy-phényl)-méthanone ;
- l'acide 4-[6-méthoxy-3-(3,4,5-triméthoxy-benzoyl)-indole-1-sulfonyl]-benzoïque ; ou
- la (5H-[1,3]dioxolo[4,5-f]indol-7-yl)-(3,4,5-triméthoxy-phényl)-méthanone.

10. Composé selon la revendication 1, dans lequel le composé est la (6-méthoxy-1H-indol-3-yl)-(3,4,5-triméthoxy-phényl)-méthanone.

11. Composé de la formule suivante : 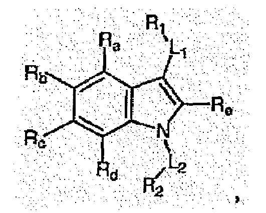 dans laquelle :
- L₁ représente C(O) ;
- L₂ représente une liaison ;
- R₁ représente aryle ou hétéroaryle à 5, 6, ou 7 chaînons trisubstitué par alkyloxy ;
- R₂ représente alkyle en C₁-C₆, alcényle en C₂-C₆, alcynyle en C₂-C₆, cyclyle ou hétérocyclyle, où chacun dudit alkyle en C₁-C₆, alcényle en C₂-C₆, alcynyle en C₂-C₆, cyclyle ou hétérocyclyle est indépendamment non substitué ou facultativement substitué par halogène, cyano, nitro, hydroxyle, amino, mercapto, alkyle, alcényle, alcynyle, aryle, hétéroaryle, cyclyle, hétérocyclyle, alkyloxy, aryloxy, alkylsulfanyle, arylsulfanyle, alkylamino, arylamino, dialkylamino, diarylamino, alkylcarbonyle, arylcarbonyle, hétéroarylcarbonyle, alkylcarboxyl,, arylcarboxyle, hétéroarylcarboxyle, alkyloxycarbonyle, aryloxycarbonyle, hétéroaryloxycarbonyle, alkylcarbamido, arylcarbamido, hétérocarbamido, alkylcarbamyle, arylcarbamyle, hétérocarbamyle ;
- Rₐ, R_{b}, R_{c} et R_{d} représentent chacun indépendamment R, halogène, nitro, isothionitro, OR, OC(O)R, OC(O)OR, OC(O)NRR', SO₂R, SO₃R, SO₂NRR', SR, NRSO₂NR'R", NRSO₂R', NRSO₃R', NRC(O)R', NRC(O)NR'R", NRC(O)OR', NRC(N)NR'R", C(O)R, C(O)OR, C(O)NRR', OP(O)(OR)(OR') O(CH₂)ₙOP(O) (OR) (OR') ou O(CH₂)ₙ-PEG, ou R_{b} et R_{c}, Rₐ et R_{b}, ou R_{c} et R_{d} pris conjointement représentent O(CH₂)ₙO ; et
- Rₑ représente H ou alkyle en C₁-C₆, où ledit alkyle en C₁-C₆ est non substitué ou facultativement substitué par halogène, cyano, nitro, hydroxyle, amino, mercapto, alkyle, alcényle, alcynyle, cyclyle, hétérocyclyle, alkyloxy, alkylsulfanyle, alkylamino, dialkylamino, alkylcarbonyle, alkylcarboxyl, alkyloxycarbonyle, alkylcarbamido, hétérocarbamido, alkylcarbamyle ou hétérocarbamyle ;
où R, R' et R" représentent chacun indépendamment H, alkyle en C₁-C₆, alcényle en C₂-C₆, alcynyle en C₂-C₆, aryle, hétéroaryle, cyclyle ou hétérocyclyle ;
- n vaut 1, 2, 3, 4 ou 5 ; et
où chacun dudit alkyle en C₁-C₆, alcényle en C₂-C₆, alcynyle en C₂-C₆, aryle, hétéroaryle, cyclyle ou hétérocyclyle est indépendamment non substitué ou facultativement substitué par halogène, cyano, nitro, hydroxyle, amino, mercapto, alkyle, alcényle, alcynyle, aryle, hétéroaryle cyclyle, hétérocyclyle, alkyloxy, aryloxy, alkylsulfanyle, arylsulfanyle, alkylamino, arylamino, dialkylamino, diarylamino, alkylcarbonyle, arylcarbonyle, hétéroarylcarbonyle, alkylcarboxyle, arylcarboxyle, hétéroarylcarboxyle, alkyloxycarbonyle, aryloxycarbonyle, hétéroaryloxycarbonyle, alkylcarbamido, arylcarbamido, hétérocarbamido, alkylcarbamyle, arylcarbamyle, hétérocarbamyle.

12. Composé selon la revendication 11, dans lequel R₁ représente 3,4,5-triméthoxyphényle.

13. Composé selon la revendication 11, dans lequel Rₑ représente H.

14. Composé selon la revendication 13, dans lequel R₁ représente 3,4,5-triméthoxyphényle.

15. Composé selon la revendication 14, dans lequel Rₐ, R_{b}, R_{c} et R_{d} représentent chacun indépendamment H, alkyloxy, alkyle en C₁-C₆ ou halogène.

16. Composé selon la revendication 15, dans lequel R_{c} représente alkyloxy, alkyle en C₁-C₆ ou halogène, et Rₐ, R_{b} et R_{d} représentent chacun H.

17. Composé selon la revendication 16 dans lequel R₂ représente alkyle en C₁-C₆, alcényle en C₂-C₆ ou alcynyle en C₂-C₆.

18. Composé selon la revendication 14, dans lequel Rₐ et R_{c} pris conjointement représentent O(CH₂)ₙO et R₁ et R_{d} représentent chacun H, où n vaut 1 ou 2.

19. Composé selon la revendication 11 dans lequel le composé est :
- la (6-méthoxy-1-pyridin-4-ylméthyl-1H-indol-3-yl)-(3,4,5-triméthoxy-phényl)-méthanone ;
- la (1-allyl-6-méthoxy-1H-indol-3-yl)-(3,4,5-triméthoxy-phényl)-méthanone ;
- la [6-méthoxy-1-(2-pipéridin-1-yl-éthyl)-1H-indol-3-yl]-(3,4,5-triméthoxy-phényl)-méthanone ;
- la (6-méthoxy-1-prop-2-ynyl-1H-indol-3-yl)-(3,4,5-triméthoxy-phényl)-méthanone ;
- la [1-(2-diméthylamino-éthyl)-5-méthoxy-1H-indol-3-yl]-(3,4,5-triméthoxy-phényl)-méthanone ;
- la (6-méthoxy-1-méthyl-1H-indol-3-yl)-(3,4,5-triméthoxy-phényl)-méthanone ;
- la [1-(2-amino-éthyl)-6-méthoxy-1H-indol-3-yl]-(3,4,5-triméthoxy-phényl)-méthanone ;
- la (1-éthyl-6-méthoxy-1H-indol-3-yl)-(3,4,5-triméthoxy-phényl)-méthanone ;
- la [6-méthoxy-1-(2-morpholin-4-yl-éthyl)-1H-indol-3-yl]-(3,4,5-triméthoxy-phényl)-méthanone ;
- la [1-(4-chloro-benzyl)-6-méthoxy-1H-indol-3-yl]-(3,4,5-triméthoxy-phényl)-méthanone ;
- la (1-benzyl-6-méthoxy-1H-indol-3-yl)-(3,4,5-triméthoxy-phényl)-méthanone ;
- la (1,6-diméthyl-1H-indol-3-yl)-(3,4,5-triméthoxy-phényl)-méthanone ;
- la (1-éthyl-6-méthyl-1H-indol-3-yl)-(3,4,5-triméthoxy-phényl)-méthanone ;
- la (1-allyl-6-méthyl-1H-indol-3-yl)-(3,4,5-triméthoxy-phényl)-méthanone ;
- la (5-éthyl-5H-[1,3]dioxolo[4,5-f]indol-7-yl)-(3,4,5-triméthoxy-phényl)-méthanone ;
- la (5-méthyl-5H-[1,3]dioxolo[4,5-f]indol-7-yl)-(3,4,5-triméthoxy-phényl)-méthanone ;
- la (5-allyl-5H-[1,3]dioxolo[4,5-f]indol-7-yl)-(3,4,5-triméthoxy-phényl)-méthanone ;
- la {6-méthoxy-1-[4-(4-nitro-phényl)-furan-2-ylméthyl]-1H-indol-3-yl}-(3,4,5-triméthoxy-phényl)-méthanone ; or
- la [1-(2-diméthylamino-éthyl)-4,5,6-triméthoxy-1H-indol-3-yl]-(3,4,5-triméthoxy-phényl)-méthanone.

20. Composé de la formule suivante : 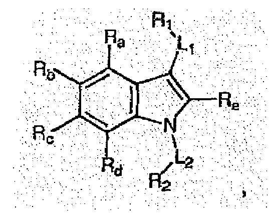 dans lequel :
- L₁ représente C(O) ;
- L₂ représente une liaison ;
- R₁ représente aryle ou hétéroaryle à 5, 6 ou 7 chaînons trisubstitué par alkyloxy ;
- R₂ représente COR"' ;
- Rₐ, R_{b}, R_{c} et R_{d} représentent chacun indépendamment R, halogène, nitro, nitroso, cyano, azide, isothionitro, OR, OC(O)R, OC(O)OR, OC(O)NRR', SO₂R, SO₃R, SO₂NRR' , SR, NRR', NRSO₂NR' R'', NRSO₂R', NRSO₃R', NRC(O)R', NRC(O)NR'R', NRC(O)OR', NRC(N)NR'R", C(O)R, C(O)OR, C(O)NRR', OP(O) (OR) (OR'), O(CH₂)ₙOP(O) (OR) (OR') ou O(CH₂)ₙ-PEG, ou R_{b} et R_{c}, Rₐ et R_{b}, ou R_{c} et R_{d} pris conjointement représentent O(CH₂)ₙO ; et
Rₑ représente H ou alkyle en C₁-C₆, où ledit alkyle en C₁-C₆ est non substitué ou facultativement substitué par halogène, cyano, nitro, hydroxyle, amino, mercapto, alkyle, alcényle, alcynyle, cyclyle, hétérocyclyle, alkyloxy, alkylsulfanyle, alkylamino, dialkylamino, alkylcarbonyle, alkylcarboxyle, alkyloxycarbonyle, alkylcarbamido, hétérocarbamido, alkylcarbamyl ou hétérocarbamyle ;
où R, R', et R" représentent indépendamment H, alkyle en C₁-C₆, alcényle en C₂-C₆, alcynyle en C₂-C₆, aryle, hétéroaryle, cyclyle ou hétérocyclyle ;
- n vaut 1, 2, 3, 4 ou 5 ; et
où chacun dudit alkyle en C₁-C₆, alcényle en C₂-C₆, alcynyle en C₂-C₆, aryle, hétéroaryle, cyclyle ou hétérocyclyle est indépendamment non substitué ou facultativement substitué par halogène, cyano, nitro, hydroxyle, amino, mercapto, alkyle, alcényle, alcynyle, aryle, hétéroaryle cyclyle, hétérocyclyle, alkyloxy, aryloxy, alkylsulfanyle, arylsulfanyle, alkylamino, arylamino, dialkylamino, diarylamino, alkylcarbonyle, arylcarbonyle, hétéroarylcarbonyle, alkylcarboxyl, arylcarboxyle, hétéroarylcarboxyle, alkyloxycarbonyle, aryloxycarbonyle, hétéroaryloxycarbonyle, alkylcarbamido, arylcarbamido, hétérocarbamido, alkylcarbamyle, arylcarbamyle, hétérocarbamyle.

21. Composé selon la revendication 20, dans lequel R₁ représente 3,4,5-triméthoxyphényle.

22. Composé selon la revendication 20, dans lequel Rₑ représente H.

23. Composé selon la revendication 22, dans lequel R₁ représente 3,4,5-triméthoxyphényle.

24. Composé selon la revendication 23, dans lequel Rₐ, R_{b}, R_{c} et R_{d} représentent chacun indépendamment H, alkyloxy, alkyl en C₁-C₆ ou halogène.

25. Composé selon la revendication 24, dans lequel R_{c} représente alkyloxy, alkyle en C₁-C₆ ou halogène, et Rₐ, R_{b} et R_{d} représentent chacun H.

26. Composé selon la revendication 25, dans lequel R''' représente alkyle en C₁-C₆, alcényle en C₂-C₆, hétérocyclyle ou hétéroaryle.

27. Composé selon la revendication 20, dans lequel le composé représente :
- la [6-méthoxy-1-(pyridine-2-carbonyl)-1H-indol-3-yl]-(3,4,5-triméthoxy-phényl)-méthanone ;
- la [6-méthoxy-1-(morpholine-4-carbonyl)-1H-indol-3-yl]-(3,4,5-triméthoxy-phényl)-méthanone ;
- la 1-[6-méthoxy-3-(3,4,5-triméthoxy-benzoyl)-indol-1-yl]-3-phényl-propénone ;
- la [1-(furan-2-carbonyl)-6-méthoxy-1H-indol-3-yl]-(3,4,5-triméthoxy-phényl)-méthanone ;
- l'ester 9H-fluorén-9-yl-méthylique de l'acide {(2-[6-méthoxy-3-(3,4,5-triméthoxy-benzoyl)-indol-1-yl]-2-oxo-éthyl}-carbamique ;
- la [6-méthoxy-1-(pyridine-3-carbonyl)-1H-indol-3-yl]-(3,4,5-triméthoxy-phényl)-méthanone ; ou
- la [6-méthoxy-1-(thiophène-2-carbonyl)-1H-indol-3-yl]-(3,4,5-triméthoxy-phényl)-méthanone.

28. Composé qui est l'un parmi :
- la (3,5-diméthoxy-phényl)-(6-méthoxy-1H-indol-3-yl)-méthanone ; et
- la (3,4-diméthoxy-phényl)-(6-méthoxy-1H-indol-3-yl)-méthanone.

29. Composé selon l'une des revendications 1, 11, 20 ou 28 destiné à être utilisé dans le traitement du cancer.

30. Composé selon l'une des revendications 1, 11, 20 et 28 destiné à être utilisé dans l'inhibition de la polymérisation de la tubuline dans un sujet en ayant besoin.

31. Composé selon l'une des revendications 1, 11, 20 ou 28 destiné à être utilisé dans le traitement d'un trouble se rapportant à l'angiogenèse.

32. Composé selon l'une des revendications 9, 10, 19, ou 27 destiné à être utilisé dans la fabrication d'un médicament pour le traitement du cancer.
